(19) Europäisches Patentamt
European Patent Office
Office européen des brevets

(11) EP 3 904 485 B1

(12) EUROPEAN PATENT SPECIFICATION

(45) Date of publication and mention of the grant of the patent:
**03.12.2025 Bulletin 2025/49**

(21) Application number: **19902599.0**

(22) Date of filing: **06.12.2019**

(51) International Patent Classification (IPC):
***C09K 11/06*** $^{(2006.01)}$ ***H10K 101/00*** $^{(2023.01)}$
***H10K 85/60*** $^{(2023.01)}$ ***H10K 71/00*** $^{(2023.01)}$

(52) Cooperative Patent Classification (CPC):
**C09K 11/06; H10K 71/311; H10K 85/615; H10K 85/622; H10K 85/626; H10K 85/633;**
C09K 2211/1011; C09K 2211/1014; H10K 50/11; H10K 2101/90

(86) International application number:
**PCT/JP2019/047801**

(87) International publication number:
**WO 2020/137443 (02.07.2020 Gazette 2020/27)**

# (54) COMPOSITION FOR LUMINESCENT ELEMENT, AND LUMINESCENT ELEMENT CONTAINING SAME

ZUSAMMENSETZUNG FÜR EIN LUMINESZIERENDES ELEMENT UND LUMINESZIERENDES ELEMENT DAMIT

COMPOSITION POUR ÉLÉMENT LUMINESCENT ET ÉLÉMENT LUMINESCENT LA CONTENANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.12.2018 JP 2018247462**

(43) Date of publication of application:
**03.11.2021 Bulletin 2021/44**

(73) Proprietor: **SUMITOMO CHEMICAL COMPANY LIMITED**
**Chuo-ku**
**Tokyo 104-8260 (JP)**

(72) Inventors:
- **SASADA, Toshiaki**
**Tsukuba-shi, Ibaraki 300-3294 (JP)**
- **MATSUMOTO, Ryuji**
**Tsukuba-shi, Ibaraki 300-3294 (JP)**

(74) Representative: **J A Kemp LLP**
**80 Turnmill Street**
**London EC1M 5QU (GB)**

(56) References cited:
**EP-A1- 1 484 382**
**EP-A2- 1 087 448**
**WO-A1-2017/130977**
**WO-A1-2017/170313**
**WO-A1-2017/170313**
**WO-A1-2018/061421**
**WO-A2-2008/063466**
**JP-A- 2001 189 192**
**JP-A- 2005 041 982**
**JP-A- 2012 042 411**
**US-A1- 2008 113 468**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 904 485 B1

## Description

Technical Field

The present invention relates to a composition for light emitting device and a light emitting device containing the same.

Background Art

Light emitting devices such as an organic electroluminescent device and the like can be suitably used, for example, for display and illumination. For example, Patent Document 1 suggests a composition containing a compound H0 and a compound EM1, as the material used for light emitting devices.

[Chemical Formula 1]

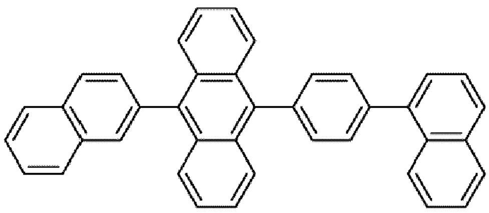

compound H0

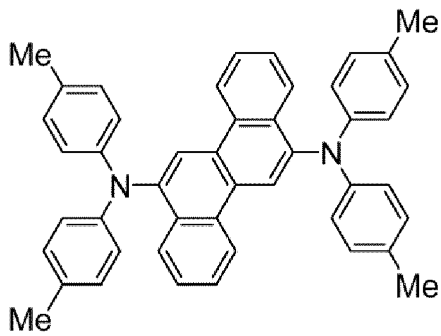

compound EM1

Patent Document 2 relates to electronic devices comprising organic semiconductors whose content of halogens is less than 20 ppm.

[Prior Art Document]

[Patent Document]

[Patent Document 1] International Publication WO2017/170313
[Patent Document 2] US 2008/113468

Summary of the Invention

Problem to be Solved by the Invention

However, a light emitting device fabricated using the composition described above was not necessarily sufficient in suppression of initial deterioration.

Then, the present invention has an object of providing a composition which is useful for production of a light emitting device of which initial deterioration is suppressed, and a light emitting device formed using the composition.

Means for Solving the Problem

The present inventors have intensively studied to solve the above-described problem and resultantly found that a sodium atom exerts a significant influence on the initial deterioration of a light emitting device having an organic layer containing a specific composition, and further found that the initial deterioration of the light emitting device can be suppressed by setting the amount of sodium atoms in a specific range, leading to completion of the present invention. However, Patent Document 1 does not describe that the amount of sodium atoms contained in a composition exerts an influence on the initial deterioration of a light emitting device.

That is, the present invention provides the following [1] to [13].

[1] A composition for light emitting device containing a host material and a guest material blended therein, wherein

the above-described host material contains an aromatic compound having a condensed ring skeleton in which only three or more benzene rings are condensed,
the above-described guest material contains an aromatic amine compound, and
the total amount of sodium atoms contained in the above-described host material and sodium atoms contained in the above-described guest material is 400 ppb by mass or less with respect to the total amount of the above-described host material and the above-described guest material; and wherein:

(a) the above-described aromatic compound is a compound represented by the formula (FH):

[Chemical Formula 2]

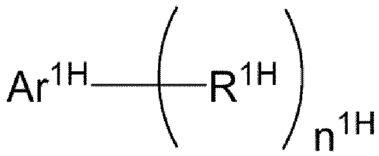
(FH)

wherein,

$n^{1H}$ represents an integer of 0 or more.
$Ar^{1H}$ represents a group obtained by removing from an aromatic hydrocarbon having a condensed ring skeleton in which only three or more benzene rings are condensed $n^{1H}$ or more hydrogen atoms bonding directly to carbon atoms constituting the above-described condensed ring, and this group optionally has a substituent. When a plurality of the substituents are present, they may be the same or different and may be combined together to form a ring together with atoms to which they are attached.
$R^{1H}$ represents an aryl group or a monovalent heterocyclic group, and these groups optionally have a substituent. When a plurality of the substituents are present, they may be the same or different and may be combined together to form a ring together with atoms to which they are attached. When a plurality of $R^{1H}$ are present, they may be the same or different and may be combined together to form a ring together with atoms to which they are attached; and

(b) the above-described aromatic amine compound is a compound represented by the formula (FB):

[Chemical Formula 3]

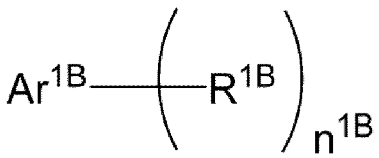
(FB)

wherein,

$n^{1B}$ represents an integer of 1 or more.
$Ar^{1B}$ represents an aromatic hydrocarbon group or an aromatic heterocyclic group, and these groups optionally have a substituent. When a plurality of the substituents are present, they may be the same or different and may be combined together to form a ring together with atoms to which they are attached.
$R^{1B}$ represents an amino group or a substituted amino group, and these groups optionally have a substituent. When a plurality of the substituents are present, they may be the same or different and may be combined together to form a ring together with atoms to which they are attached. When a plurality of $R^{1B}$ are present, they may be the same or different and may be combined together to form a ring together with atoms to which they are attached.

[2] The composition for light emitting device according to [1], wherein the above-described condensed ring skeleton is a condensed ring skeleton in which only three or more and five or less benzene rings are condensed.
[3] The composition for light emitting device according to [2], wherein the above-described condensed ring skeleton is an anthracene skeleton, a phenanthrene skeleton, a benzoanthracene skeleton, a benzophenanthrene skeleton or a pyrene skeleton.
[4] The composition for light emitting device according to any one of [1] to [3], further comprising at least one selected from the group consisting of a hole transporting material, a hole injection material, an electron transporting material, an electron injection material, a light emitting material, an antioxidant and a solvent.
[5] A light emitting device having an anode, a cathode, and an organic layer disposed between the above-described anode and the above-described cathode, wherein
the above-described organic layer is a layer containing the composition for light emitting device as described in any one of [1] to [4].
[6] A method for producing a composition for light emitting device containing a host material and a guest material blended therein, comprising

a host material preparation step of preparing a host material containing an aromatic compound having a condensed ring skeleton in which only three or more benzene rings are condensed,
a guest material preparation step of preparing a guest material containing an aromatic amine compound, and
a production method of mixing the above-described host material and the above-described guest material at a blending ratio such that the total amount of sodium atoms contained in the above-described host material and sodium atoms contained in the above-described guest material is 400 ppb by mass or less to obtain a composition for light emitting device; and wherein:

(a) the above-described aromatic compound is a compound represented by the formula (FH):

[Chemical Formula 2]

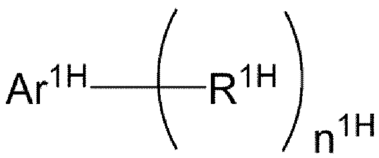
(FH)

wherein,

$n^{1H}$ represents an integer of 0 or more.
$Ar^{1H}$ represents a group obtained by removing from an aromatic hydrocarbon having a condensed ring skeleton in which only three or more benzene rings are condensed $n^{1H}$ or more hydrogen atoms bonding directly to carbon atoms constituting the above-described condensed ring, and this group optionally has a substituent. When a plurality of the substituents are present, they may be the same or different and may be combined together to form a ring together with atoms to which they are attached.
$R^{1H}$ represents an aryl group or a monovalent heterocyclic group, and these groups optionally have a substituent. When a plurality of the substituents are present, they may be the same or different and may be combined together to form a ring together with atoms to which they are attached. When a plurality of $R^{1H}$ are present, they may be the same or different and may be combined together to form a ring together with atoms to which they are attached; and

(b) the above-described aromatic amine compound is a compound represented by the formula (FB):

[Chemical Formula 3]

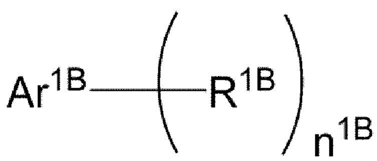
(FB)

wherein,

$n^{1B}$ represents an integer of 1 or more.

$Ar^{1B}$ represents an aromatic hydrocarbon group or an aromatic heterocyclic group, and these groups optionally have a substituent. When a plurality of the substituents are present, they may be the same or different and may be combined together to form a ring together with atoms to which they are attached.

$R^{1B}$ represents an amino group or a substituted amino group, and these groups optionally have a substituent. When a plurality of the substituents are present, they may be the same or different and may be combined together to form a ring together with atoms to which they are attached. When a plurality of $R^{1B}$ are present, they may be the same or different and may be combined together to form a ring together with atoms to which they are attached.

[7] The production method according to [6], wherein
the above-described guest material preparation step comprises

a preparation step (B-1) of preparing the above-described aromatic amine compound containing sodium atoms mixed therein, and
a step (B-2) of purifying at least a part of the above-described aromatic amine compound prepared in the above-described step (B-1) to remove at least a part of the above-described sodium atoms.

[8] The production method according to [6] or [7], wherein
the above-described host material preparation step comprises

a step (A-1) of preparing the above-described aromatic compound containing sodium atoms mixed therein, and
a step (A-2) of purifying at least a part of the above-described aromatic compound prepared in the above-described step (A-1) to remove at least a part of the above-described sodium atoms.

[9] A method for producing a composition for light emitting device containing a host material and a guest material blended therein, comprising

a host material preparation step of preparing a host material containing an aromatic compound having a condensed ring skeleton in which only three or more benzene rings are condensed,
a determination step of determining the blending ratio of the guest material to the above-described host material,
a guest material preparation step of preparing a guest material which contains an aromatic amine compound and with which, when mixed with the above-described host material at the above-described blending ratio, the total amount of sodium atoms contained in the above-described host material and sodium atoms contained in the above-described guest material with respect to the total amount of the above-described host material and the above-described guest material is 400 ppb by mass or less, and
a production step of mixing the above-described host material and the above-described guest material at the above-described blending ratio to obtain a composition for light emitting device; and wherein:

(a) the above-described aromatic compound is a compound represented by the formula (FH):

[Chemical Formula 2]

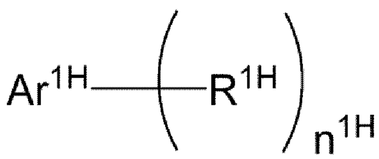

(FH)

wherein,

$n^{1H}$ represents an integer of 0 or more.

$Ar^{1H}$ represents a group obtained by removing from an aromatic hydrocarbon having a condensed ring skeleton in which only three or more benzene rings are condensed $n^{1H}$ or more hydrogen atoms bonding directly to carbon atoms constituting the above-described condensed ring, and this group optionally has

a substituent. When a plurality of the substituents are present, they may be the same or different and may be combined together to form a ring together with atoms to which they are attached.

$R^{1H}$ represents an aryl group or a monovalent heterocyclic group, and these groups optionally have a substituent. When a plurality of the substituents are present, they may be the same or different and may be combined together to form a ring together with atoms to which they are attached. When a plurality of $R^{1H}$ are present, they may be the same or different and may be combined together to form a ring together with atoms to which they are attached; and

(b) the above-described aromatic amine compound is a compound represented by the formula (FB):

[Chemical Formula 3]

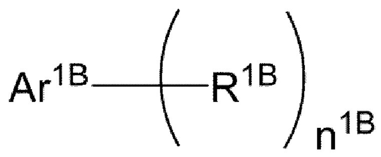

(FB)

wherein,

$n^{1B}$ represents an integer of 1 or more.

$Ar^{1B}$ represents an aromatic hydrocarbon group or an aromatic heterocyclic group, and these groups optionally have a substituent. When a plurality of the substituents are present, they may be the same or different and may be combined together to form a ring together with atoms to which they are attached.

$R^{1B}$ represents an amino group or a substituted amino group, and these groups optionally have a substituent. When a plurality of the substituents are present, they may be the same or different and may be combined together to form a ring together with atoms to which they are attached. When a plurality of $R^{1B}$ are present, they may be the same or different and may be combined together to form a ring together with atoms to which they are attached.

[10] A method for producing a composition for light emitting device containing a host material and a guest material blended therein, comprising

a guest material preparation step of preparing a guest material containing an aromatic amine compound,
a determination step of determining the blending ratio of the host material to the above-described guest material,
a host material preparation step of preparing a host material which contains an aromatic compound having a condensed ring skeleton in which only three or more benzene rings are condensed and with which, when mixed with the above-described guest material at the above-described blending ratio, the total amount of sodium atoms contained in the above-described host material and sodium atoms contained in the above-described guest material with respect to the total amount of the above-described host material and the above-described guest material is 400 ppb by mass or less, and
a production step of mixing the above-described guest material and the above-described host material at the above-described blending ratio to obtain a composition for light emitting device; and wherein:

(a) the above-described aromatic compound is a compound represented by the formula (FH):

[Chemical Formula 2]

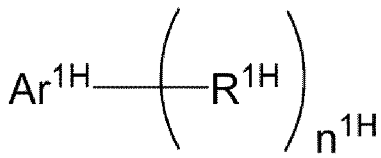

(FH)

wherein,

$n^{1H}$ represents an integer of 0 or more.

$Ar^{1H}$ represents a group obtained by removing from an aromatic hydrocarbon having a condensed ring skeleton in which only three or more benzene rings are condensed $n^{1H}$ or more hydrogen atoms bonding directly to carbon atoms constituting the above-described condensed ring, and this group optionally has a substituent. When a plurality of the substituents are present, they may be the same or different and may be combined together to form a ring together with atoms to which they are attached.

$R^{1H}$ represents an aryl group or a monovalent heterocyclic group, and these groups optionally have a substituent. When a plurality of the substituents are present, they may be the same or different and may be combined together to form a ring together with atoms to which they are attached. When a plurality of $R^{1H}$ are present, they may be the same or different and may be combined together to form a ring together with atoms to which they are attached; and

(b) the above-described aromatic amine compound is a compound represented by the formula (FB):

[Chemical Formula 3]

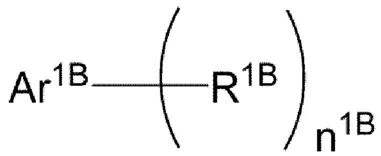

(FB)

wherein,

$n^{1B}$ represents an integer of 1 or more.

$Ar^{1B}$ represents an aromatic hydrocarbon group or an aromatic heterocyclic group, and these groups optionally have a substituent. When a plurality of the substituents are present, they may be the same or different and may be combined together to form a ring together with atoms to which they are attached.

$R^{1B}$ represents an amino group or a substituted amino group, and these groups optionally have a substituent. When a plurality of the substituents are present, they may be the same or different and may be combined together to form a ring together with atoms to which they are attached. When a plurality of $R^{1B}$ are present, they may be the same or different and may be combined together to form a ring together with atoms to which they are attached.

[11] A method for producing a composition for light emitting device containing a host material and a guest material blended therein, comprising

a host material preparation step of preparing an aromatic compound having a condensed ring skeleton in which only three or more benzene rings are condensed as the host material,

a guest material preparation step of preparing an aromatic amine compound as the guest material,

a determination step of determining the blending ratio of the above-described host material and the above-described guest material,

a purification step of purifying at least a part of the above-described aromatic compound and the above-described aromatic amine compound such that, when the above-described host material and the above-described guest material are mixed at the above-described blending ratio, the total amount of sodium atoms contained in the above-described host material and sodium atoms contained in the above-described guest material with respect to the total amount of the above-described host material and the above-described guest material is 400 ppb by mass or less, and

a production step of mixing the above-described host material containing the above-described aromatic compound and the above-described guest material containing the above-described aromatic amine compound at the above-described blending ratio to obtain a composition for light emitting device; and wherein:

(a) the above-described aromatic compound is a compound represented by the formula (FH):

[Chemical Formula 2]

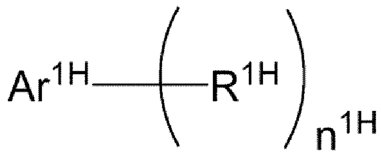 (FH)

wherein,

$n^{1H}$ represents an integer of 0 or more.
$Ar^{1H}$ represents a group obtained by removing from an aromatic hydrocarbon having a condensed ring skeleton in which only three or more benzene rings are condensed $n^{1H}$ or more hydrogen atoms bonding directly to carbon atoms constituting the above-described condensed ring, and this group optionally has a substituent. When a plurality of the substituents are present, they may be the same or different and may be combined together to form a ring together with atoms to which they are attached.
$R^{1H}$ represents an aryl group or a monovalent heterocyclic group, and these groups optionally have a substituent. When a plurality of the substituents are present, they may be the same or different and may be combined together to form a ring together with atoms to which they are attached. When a plurality of $R^{1H}$ are present, they may be the same or different and may be combined together to form a ring together with atoms to which they are attached; and

(b) the above-described aromatic amine compound is a compound represented by the formula (FB):

[Chemical Formula 3]

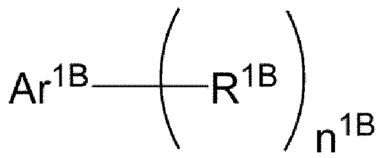 (FB)

wherein,

$n^{1B}$ represents an integer of 1 or more.
$Ar^{1B}$ represents an aromatic hydrocarbon group or an aromatic heterocyclic group, and these groups optionally have a substituent. When a plurality of the substituents are present, they may be the same or different and may be combined together to form a ring together with atoms to which they are attached.
$R^{1B}$ represents an amino group or a substituted amino group, and these groups optionally have a substituent. When a plurality of the substituents are present, they may be the same or different and may be combined together to form a ring together with atoms to which they are attached. When a plurality of $R^{1B}$ are present, they may be the same or different and may be combined together to form a ring together with atoms to which they are attached.

[12] The production method according to any one of [6] to [11], further comprising

a host material measurement step of measuring the content of sodium atoms contained in the above-described aromatic compound, and
a guest material measurement step of measuring the content of sodium atoms contained in the above-described aromatic amine compound.

[13] A method for producing a light emitting device containing an anode, a cathode, and an organic layer disposed between the above-described anode and the above-described cathode, comprising
a step of forming the above-described organic layer with the composition for light emitting device produced by the production method as described in any one of [6] to [12].

Effect of the Invention

According to the present invention, a composition which is useful for production of a light emitting device of which

initial deterioration is suppressed can be provided. In addition, according to the present invention, a light emitting device containing the above-described composition can be provided. Further, according to the present invention, methods for producing the above-described composition and the above-described light emitting device can be provided.

Modes for Carrying Out the Invention

Suitable embodiments of the present embodiment will be illustrated in detail below.

<Explanation of common terms>

The terms commonly used in the present specification have the following meanings, unless otherwise stated.
"Room temperature" means 25°C.
Me represents a methyl group, Et represents an ethyl group, Bu represents a butyl group, i-Pr represents an isopropyl group, and t-Bu represents a tert-butyl group.
The hydrogen atom may be a heavy hydrogen atom or a light hydrogen atom.
"The polymer compound" means a polymer having molecular weight distribution and having a polystyrene-equivalent number-average molecular weight of $1\times10^3$ to $1\times10^8$.
"The low-molecular compound" means a compound having no molecular weight distribution and having a molecular weight of $1\times10^4$ or less.
"The constitutional unit" means a unit occurring once or more times in a polymer compound.
"The alkyl group" may be any of linear and branched. The number of carbon atoms of the linear alkyl group, not including the number of carbon atoms of the substituent, is usually 1 to 50, preferably 1 to 20, and more preferably 1 to 10. The number of carbon atoms of the branched alkyl group, not including the number of carbon atoms of the substituent, is usually 3 to 50, preferably 3 to 20, and more preferably 4 to 10. The alkyl group optionally has a substituent, and examples thereof include a methyl group, an ethyl group, an isopropyl group, a butyl group, an isobutyl group, a tert-butyl group, a pentyl group, a hexyl group, a heptyl group, an octyl group, a 2-ethylhexyl group, a decyl group, a 3,7-dimethyloctyl group, a 2-ethyloctyl group, a dodecyl group, a trifluoromethyl group, a 3-phenylpropyl group, a 3-(4-methylphenyl)propyl group, a 3-(3,5-di-hexylphenyl)propyl group, and a 6-ethyloxyhexyl group.
The number of carbon atoms of "the cycloalkyl group", not including the number of carbon atoms of the substituent, is usually 3 to 50, and preferably 4 to 10. The cycloalkyl group optionally has a substituent, and examples thereof include a cyclohexyl group and a methylcyclohexyl group.
The number of carbon atoms of "the alkylene group", not including the number of carbon atoms of the substituent, is usually 1 or more and 20 or less, preferably 1 or more and 15 or less, and more preferably 1 or more and 10 or less. The alkylene group optionally has a substituent, and examples thereof include a methylene group, an ethylene group, a propylene group, a butylene group, a hexylene group and an octylene group.
The number of carbon atoms of "the cycloalkylene group", not including the number of carbon atoms of the substituent, is usually 3 or more and 20 or less. The cycloalkylene group optionally has a substituent, and examples thereof include a cyclohexylene group.
"The aromatic hydrocarbon group" means a group obtained by removing from an aromatic hydrocarbon one or more hydrogen atoms bonding directly to carbon atoms constituting the ring. The group obtained by removing from an aromatic hydrocarbon one hydrogen atom bonding directly to a carbon atom constituting the ring is referred to also as "aryl group". The group obtained by removing from an aromatic hydrocarbon two hydrogen atoms bonding directly to carbon atoms constituting the ring is referred to also as "arylene group".
The number of carbon atoms of the aromatic hydrocarbon group, not including the number of carbon atoms of the substituent, is usually 6 to 60, preferably 6 to 30, and more preferably 6 to 18.
"The aromatic hydrocarbon group" includes, for example, groups obtained by removing from a monocyclic aromatic hydrocarbon (including, for example, benzene) or a polycyclic aromatic hydrocarbon (including, for example, bicyclic aromatic hydrocarbons such as naphthalene and indene and the like; tricyclic aromatic hydrocarbons such as anthracene, phenanthrene, dihydrophenanthrene and fluorene and the like; tetracyclic aromatic hydrocarbons such as benzoanthracene, benzophenanthrene, benzofluorene, pyrene and fluoranthene and the like; pentacyclic aromatic hydrocarbons such as dibenzoanthracene, dibenzophenanthrene, dibenzofluorene, perylene and benzofluoranthene and the like; hexacyclic aromatic hydrocarbons such as spirobifluorene and the like; and heptacyclic aromatic hydrocarbons such as benzospirobifluorene and acenaphthofluoranthene and the like) one or more hydrogen atoms bonding directly to carbon atoms constituting the ring, and these groups optionally have a substituent. The aromatic hydrocarbon group includes groups obtained by bonding a plurality of these groups.
"The alkoxy group" may be any of linear and branched. The number of carbon atoms of the linear alkoxy group, not including the number of carbon atoms of the substituent, is usually 1 to 40, and preferably 1 to 10. The number of carbon atoms of the branched alkoxy group, not including the number of carbon atoms of the substituent, is usually 3 to 40, and

preferably 4 to 10. The alkoxy group optionally has a substituent, and examples thereof include a methoxy, an ethoxy, an isopropyloxy group, a butyloxy group, a hexyloxy group, a 2-ethylhexyloxy group, a 3,7-dimethyloctyloxy group, and a lauryloxy group.

The number of carbon atoms of "the cycloalkoxy group", not including the number of carbon atoms of the substituent, is usually 3 to 40, and preferably 4 to 10. The cycloalkoxy group optionally has a substituent, and examples thereof include a cyclohexyloxy group.

The number of carbon atoms of "the aryloxy group", not including the number of carbon atoms of the substituent, is usually 6 to 60, and preferably 6 to 48. The aryloxy group optionally has a substituent, and examples thereof include a phenoxy group, a naphthyloxy group, an anthracenyloxy group, and a pyrenyloxy group.

"The heterocyclic group" means a group obtained by removing from a heterocyclic compound one or more hydrogen atoms bonding directly to carbon atoms or hetero atoms constituting the ring. Of heterocyclic groups, "aromatic heterocyclic group" which is a group obtained by removing from an aromatic heterocyclic compound one or more hydrogen atoms bonding directly to carbon atoms or hetero atoms constituting the ring is preferable. A group obtained by removing from a heterocyclic compound p hydrogen atoms (p represents an integer of 1 or more) bonding directly to carbon atoms or hetero atoms constituting the ring is referred to also as "p-valent heterocyclic group". A group obtained by removing from an aromatic heterocyclic compound p hydrogen atoms bonding directly to carbon atoms or hetero atoms constituting the ring is referred to also as "p-valent aromatic heterocyclic group".

"The aromatic heterocyclic compound" includes, for example, compounds of which heterocyclic ring itself shows aromaticity such as azole, thiophene, furan, pyridine, diazabenzene, triazine, azanaphthalene, diazanaphthalene and carbazole and the like, and compounds in which an aromatic ring is condensed to the heterocyclic ring even if the heterocyclic ring itself shows no aromaticity such as phenoxazine, phenothiazine and benzopyran and the like.

The number of carbon atoms of the heterocyclic group, not including the number of carbon atoms of the substituent, is usually 1 to 60, preferably 2 to 40, and more preferably 3 to 20. The number of hetero atoms of the heterocyclic group, not including the number of carbon atoms of the substituent, is usually 1 to 30, preferably, 1 to 10, and more preferably 1 to 3.

The heterocyclic group optionally has a substituent, and examples thereof include groups obtained by removing from a monocyclic heterocyclic compound (including, for example, furan, thiophene, oxadiazole, pyrrole, diazole, triazole, tetrazole, pyridine, diazabenzene and triazine) or a polycyclic heterocyclic compound (including, for example, bicyclic heterocyclic compounds such as azanaphthalene, diazanaphthalene, benzofuran, benzothiophene, indole, benzodiazole and benzothiadiazole and the like; tricyclic heterocyclic compounds such as dibenzofuran, dibenzothiophene, dibenzoborole, dibenzosilole, dibenzophoshole, dibenzoselenophene, carbazole, azacarbazole, diazacarbazole, phenoxazine, phenothiazine, 9,10-dihydroacridine, 5,10-dihydrophenazine, phenazaborine, phenophosphazine, phenoselenazine, phenazasiline, azaanthracene, diazaanthracene, azaphenanthrene and diazaphenanthrene and the like; tetracyclic heterocyclic compounds such as hexaazatriphenylene, benzocarbazole, benzonaphthofuran and benzonaphthothiophene and the like; pentacyclic heterocyclic compounds such as dibenzocarbazole, indolocarbazole and indenocarbazole and the like; hexacyclic heterocyclic compounds such as carbazolocarbazole, benzoindolocarbazole and benzoindenocarbazole and the like; and heptacyclic heterocyclic compounds such as dibenzoindolocarbazole and the like) one or more hydrogen atoms bonding directly to atoms constituting the ring, and these groups optionally have a substituent. The heterocyclic group includes groups obtained by bonding a plurality of these groups.

"The halogen atom" denotes a fluorine atom, a chlorine atom, a bromine atom or an iodine atom.

"The amino group" optionally has a substituent, with substituted amino groups (namely, secondary amino groups or tertiary amino groups, and preferably tertiary amino groups) being preferred. As the substituent which an amino group has, an alkyl group, a cycloalkyl group, an aryl group or a monovalent heterocyclic group is preferable. When a plurality of the substituents which an amino group has are present, they may be the same or different and may be combined together to form a ring together with nitrogen atoms to which they are attached.

The substituted amino group includes, for example, a dialkylamino group, a dicycloalkylamino group and a diarylamino group.

The amino group includes, for example, a dimethylamino group, a diethylamino group, a diphenylamino group, a bis(methylphenyl)amino group and a bis(3,5-di-tert-butylphenyl)amino group.

"The alkenyl group" may be any of linear and branched. The number of carbon atoms of the linear alkenyl group, not including the number of carbon atoms of the substituent, is usually 2 to 30, and preferably 3 to 20. The number of carbon atoms of the branched alkenyl group, not including the number of carbon atoms of the substituent, is usually 3 to 30, and preferably 4 to 20.

The number of carbon atoms of "the cycloalkenyl group", not including the number of carbon atoms of the substituent, is usually 3 to 30, and preferably 4 to 20.

The alkenyl group and the cycloalkenyl group optionally have a substituent, and examples thereof include a vinyl group, a propenyl group, a butenyl group, a 3-butenyl group, a 3-pentenyl group, a 4-pentenyl group, a 1-hexenyl group, a 5-hexenyl group, a 7-octenyl group, and these groups having a substituent.

"The alkynyl group" may be any of linear and branched. The number of carbon atoms of the alkynyl group, not including carbon atoms of the substituent, is usually 2 to 20, and preferably 3 to 20. The number of carbon atoms of the branched alkynyl group, not including carbon atoms of the substituent, is usually 4 to 30, and preferably 4 to 20.

The number of carbon atoms of "the cycloalkynyl group", not including carbon atoms of the substituent, is usually 4 to 30, and preferably 4 to 20.

The alkynyl group and the cycloalkynyl group optionally have a substituent, and examples thereof include an ethynyl group, a propynyl group, a butynyl group, a pentynyl group, a hexynyl group, a 5-hexynyl group, and these groups having a substituent.

"The crosslinkable group" is a group which is capable of generating a new bond by being substituted to heating, ultraviolet irradiation, near ultraviolet irradiation, visible light irradiation, infrared irradiation, radical reaction and the like, and is preferably a group represented by any of the formula (B-1) to the formula (B-17). These groups optionally have a substituent.

[Chemical Formula 4]

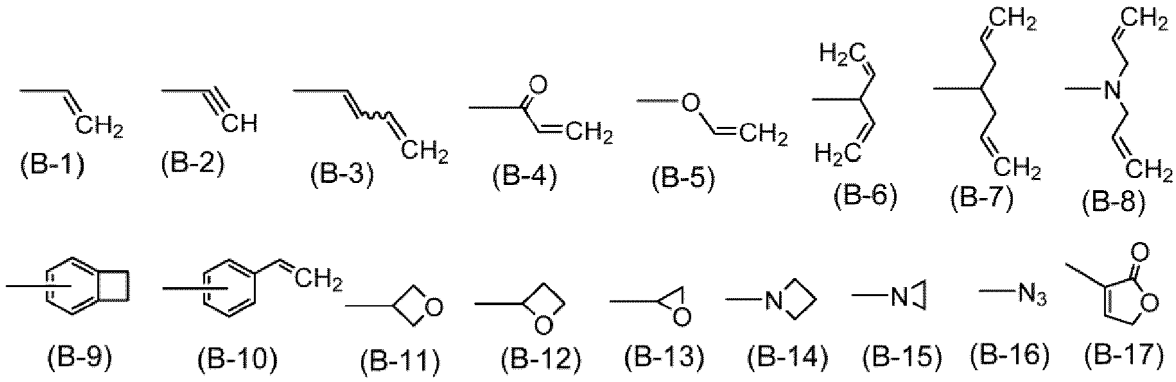

(B-1) (B-2) (B-3) (B-4) (B-5) (B-6) (B-7) (B-8)

(B-9) (B-10) (B-11) (B-12) (B-13) (B-14) (B-15) (B-16) (B-17)

"The substituent" includes, for example, a halogen atom, a cyano group, an alkyl group, a cycloalkyl group, an aryl group, a monovalent heterocyclic group, an alkoxy group, a cycloalkoxy group, an aryloxy group, an amino group, a substituted amino group, an alkenyl group, a cycloalkenyl group, an alkynyl group and a cycloalkynyl group. The substituent may be a crosslinkable group. When a plurality of substituents are present, they may be combined together to form a ring together with atoms to which they are attached, but it is preferable that they do not form a ring.

"The amount of sodium atoms" can be measured by an ICP/MS method (inductively coupled plasma mass spectrometry). That is, "the amount of sodium atoms" means the mass concentration of sodium atoms when measured by an ICP/MS method. Further, when "the amount of sodium atoms" is "0 ppb by mass", it means that the mass concentration of sodium atoms is below the detection limit when measured by an ICP/MS method.

In the composition for light emitting device of the present embodiment, the host material means a material that physically, chemically or electrically interacts with a guest material. By this interaction, for example, the light emitting property, the charge transporting property or the charge injection property of the composition for light emitting device of the present embodiment can be improved or adjusted.

In the composition for light emitting device of the present embodiment, when the light emitting material is explained as an example, the guest material can be made to emit light more efficiently by electrically interacting with the host material and efficiently transferring electrical energy from the host material to the guest material.

<Host material>

The host material contains an aromatic compound having a condensed ring skeleton in which only three or more benzene rings are condensed. The aromatic compound having a condensed ring skeleton in which only three or more benzene rings are condensed may contain only one type of the condensed ring skeleton in which only three or more benzene rings are condensed or may contain two or more types thereof, in the compound. Further, the aromatic compound having a condensed ring skeleton in which only three or more benzene rings are condensed may contain only one condensed ring skeleton in which only three or more benzene rings are condensed or may contain two or more condensed ring skeletons, in the compound. Hereinafter, the aromatic compound having a condensed ring skeleton in which only three or more benzene rings are condensed contained in the host material is referred to as "aromatic compound for host material" in some cases.

In the condensed ring skeleton which the aromatic compound for host material has, the number of the condensed benzene rings is usually 3 to 10, and it is preferably 3 to 7, more preferably 3 to 5 and further preferably 3 or 4 since the initial

deterioration of the light emitting device of the present embodiment is more suppressed.

The condensed ring skeleton which the aromatic compound for host material has can also be said to be a carbon skeleton of a condensed ring in which only three or more benzene rings are condensed. The condensed ring skeleton is preferably an anthracene skeleton, a phenanthrene skeleton, a benzoanthracene skeleton, a benzophenanthrene skeleton or a pyrene skeleton, more preferably an anthracene skeleton, a benzoanthracene skeleton or a pyrene skeleton and further preferably an anthracene skeleton since the initial deterioration of the light emitting device of the present embodiment is more suppressed.

The aromatic compound for host material may be an aromatic hydrocarbon having a condensed ring skeleton in which only three or more benzene rings are condensed (hereinafter, referred to also as "condensed ring-containing aromatic hydrocarbon"), and the aromatic hydrocarbon optionally has a substituent. The substituent which the condensed ring-containing aromatic hydrocarbon optionally has is preferably a halogen atom, an alkyl group, a cycloalkyl group, an alkoxy group, a cycloalkoxy group, an aryl group, a monovalent heterocyclic group or a substituted amino group, more preferably an alkyl group, a cycloalkyl group, an aryl group or a monovalent heterocyclic group, further preferably an aryl group or a monovalent heterocyclic group, and particularly preferably an aryl group, and these groups optionally further have a substituent.

The aryl group as the substituent which the condensed ring-containing aromatic hydrocarbon optionally has is preferably a group obtained by removing from a monocyclic or bicyclic to hexacyclic aromatic hydrocarbon one or more hydrogen atoms bonding directly to carbon atoms constituting the ring, more preferably a group obtained by removing from a monocyclic or bicyclic to tetracyclic aromatic hydrocarbon one or more hydrogen atoms bonding directly to carbon atoms constituting the ring, further preferably a group obtained by removing from benzene, naphthalene, dihydrophenanthrene, fluorene or benzofluorene one or more hydrogen atoms bonding directly to carbon atoms constituting the ring, and particularly preferably a phenyl group or a naphthyl group, and these groups optionally have a substituent.

The monovalent heterocyclic group as the substituent which the condensed ring-containing aromatic hydrocarbon optionally has is preferably a group obtained by removing from a monocyclic or bicyclic to hexacyclic heterocyclic compound one or more hydrogen atoms bonding directly to atoms constituting the ring, more preferably a group obtained by removing from a monocyclic or bicyclic to tetracyclic heterocyclic compound one or more hydrogen atoms bonding directly to atoms constituting the ring, and further preferably a group obtained by removing from pyridine, diazabenzene, triazine, azanaphthalene, diazanaphthalene, dibenzofuran, dibenzothiophene, carbazole, benzocarbazole, benzonaphthofuran or benzonaphthothiophene one or more hydrogen atoms bonding directly to atoms constituting the ring, and these groups optionally have a substituent.

In the substituted amino group as the substituent which the condensed ring-containing aromatic hydrocarbon optionally has, the substituent which an amino group has is preferably an aryl group or a monovalent heterocyclic group, more preferably an aryl group, and these groups optionally further have a substituent. The examples and the preferable range of the aryl group as the substituent which an amino group has are the same as the examples and the preferable range of the aryl group as the substituent which the condensed ring-containing aromatic hydrocarbon optionally has. The examples and the preferable range of the monovalent heterocyclic group as the substituent which an amino group has are the same as the examples and the preferable range of the monovalent heterocyclic group as the substituent which the condensed ring-containing aromatic hydrocarbon optionally has.

It is preferable that the condensed ring-containing aromatic hydrocarbon has a substituent, since the initial deterioration of the light emitting device of the present embodiment is more suppressed. When the condensed ring-containing aromatic hydrocarbon has a substituent, the total number of the substituents which the condensed ring-containing aromatic hydrocarbon has is usually 1 to 20 (provided that, it is not higher than the total number of hydrogen atoms which the condensed ring-containing aromatic hydrocarbon has, the same shall apply hereinafter), and it is preferably 1 to 15, more preferably 1 to 10, further preferably 1 to 7, particularly preferably 1 to 5 and especially preferably 1 to 3 since synthesis of the host material is easy.

The substituent which the substituent which the condensed ring-containing aromatic hydrocarbon optionally has optionally further has is preferably a halogen atom, an alkyl group, a cycloalkyl group, an alkoxy group, a cycloalkoxy group, an aryl group, a monovalent heterocyclic group or a substituted amino group, more preferably an alkyl group, a cycloalkyl group, an aryl group, a monovalent heterocyclic group or a substituted amino group, and further preferably an alkyl group or a cycloalkyl group, and these groups optionally further have a substituent, but it is preferable that they do not further have a substituent.

The examples and the preferable range of the aryl group, the monovalent heterocyclic group and the substituted amino group as the substituent which the substituent which the condensed ring-containing aromatic hydrocarbon optionally has optionally further has are the same as the examples and the preferable range of the aryl group, the monovalent heterocyclic group and the substituted amino group as the substituent which the condensed ring-containing aromatic hydrocarbon optionally has, respectively.

The host material may further contain the compound other than the aromatic compound for host material, however, it is preferable that the aromatic compound for host material is contained as the main component since the initial

deterioration of the light emitting device of the present embodiment is more suppressed. The content ratio of the aromatic compound for host material occupying the host material may be, for example, 10% by mass or more, and it is preferably 30% by mass or more, more preferably 50% by mass or more, further preferably 70% by mass or more, particularly preferably 90% by mass or more and especially preferably 95% by mass or more and may also be 100% by mass since the initial deterioration of the light emitting device of the present embodiment is more suppressed.

The host material may contain only one type of the aromatic compound for host material or may contain two or more types thereof. When the host material further contains a compound other than the aromatic compound for host material, the host material may contain only one type of the compound other than the aromatic compound for host material or may contain two or more types thereof.

In general, an aromatic compound for host material may be a polymer compound (hereinafter, referred to also as "polymer host material") or a low-molecular compound (hereinafter, referred to also as "low-molecular host material"), with the low-molecular host material being preferred. In the presently claimed invention, the host material specifically contains at least an aromatic compound for host material that is a compound represented by the formula (FH).

(Low-molecular host material)

The molecular weight of the low-molecular host material is usually $1\times10^2$ to $1\times10^4$, preferably $2\times10^2$ to $5\times10^3$, more preferably $3\times10^2$ to $2\times10^3$ and further preferably $4\times10^2$ to $1\times10^3$.

The total number of the condensed ring skeletons in which only three or more benzene rings are condensed contained in the low-molecular host material is usually 1 to 10, and it is preferably 1 to 7, more preferably 1 to 5, further preferably 1 to 3 and particularly preferably 1 since the initial deterioration of the light emitting device of the present embodiment is more suppressed.

The low-molecular host material may contain only one type of the condensed ring skeleton in which only three or more benzene rings are condensed or may contain two or more types thereof, and the number of the types is preferably 1 to 5, more preferably 1 to 3 and further preferably 1 since synthesis of the low-molecular host material is easy.

Since the initial deterioration of the light emitting device of the present embodiment is more suppressed, it is preferable that the low-molecular host material contains a condensed ring skeleton in which only three or more benzene rings are condensed, as a group obtained by removing from an aromatic hydrocarbon having a condensed ring skeleton in which only three or more benzene rings are condensed one or more hydrogen atoms bonding directly to carbon atoms constituting the condensed ring (hereinafter, referred to also as "condensed ring-containing aromatic hydrocarbon group"), and this group optionally has a substituent.

The total number of the condensed ring-containing aromatic hydrocarbon groups contained in the low-molecular host material is usually 1 to 10, and it is preferably 1 to 7, more preferably 1 to 5, further preferably 1 to 3 and particularly preferably 1 since the initial deterioration of the light emitting device of the present embodiment is more suppressed.

The low-molecular host material may contain only one type of the condensed ring-containing aromatic hydrocarbon group or may contain two or more types thereof, and the number of the types is preferably 1 to 5, more preferably 1 to 3 and further preferably 1 since synthesis of the low-molecular host material is easy.

In the low-molecular host material, the examples and the preferable range of the substituent which the condensed ring-containing aromatic hydrocarbon group optionally has are the same as the examples and the preferable range of the substituent which the condensed ring-containing aromatic hydrocarbon optionally has.

[Compound represented by the formula (FH)]

In the claimed invention, the host material contains at least a low-molecular host material that is a compound represented by the formula (FH), since the initial deterioration of the light emitting device of the present embodiment is more suppressed.

$n^{1H}$ is usually an integer of 10 or less, and it is preferably an integer of 7 or less, more preferably an integer of 5 or less and further preferably an integer of 3 or less since synthesis of a compound represented by the formula (FH) is easy. Further, $n^{1H}$ is preferably an integer of 1 or more, since the initial deterioration of the light emitting device of the present embodiment is more suppressed.

The substituent which the condensed ring-containing aromatic hydrocarbon group represented by $Ar^{1H}$ optionally has is a substituent other than an aryl group and a monovalent heterocyclic group, and it is preferably a halogen atom, an alkyl group, a cycloalkyl group, an alkoxy group, a cycloalkoxy group or a substituted amino group and more preferably an alkyl group or a cycloalkyl group, and these groups optionally further have a substituent.

The examples and the preferable range of the substituted amino group as the substituent which the condensed ring-containing aromatic hydrocarbon group optionally has are the same as the examples and the preferable range of the substituted amino group as the substituent which the condensed ring-containing aromatic hydrocarbon optionally has.

The examples and the preferable range of the substituent which the substituent which the condensed ring-containing aromatic hydrocarbon group optionally has optionally further has are the same as the examples and the preferable range of the substituent which the substituent which the condensed ring-containing aromatic hydrocarbon optionally has optionally further has.

$R^{1H}$ is preferably an aryl group optionally having a substituent, since the initial deterioration of the light emitting device of the present embodiment is more suppressed.

The examples and the preferable range of the aryl group and the monovalent heterocyclic group represented by $R^{1H}$ are the same as the examples and the preferable range of the aryl group and the monovalent heterocyclic group as the substituent which the condensed ring-containing aromatic hydrocarbon optionally has, respectively.

The examples and the preferable range of the substituent which $R^{1H}$ optionally has are the same as the examples and the preferable range of the substituent which the substituent which the condensed ring-containing aromatic hydrocarbon optionally has optionally further has.

As the low-molecular host material, compounds represented by the following formulae and compounds described in examples are exemplified. These compounds optionally have a substituent. In the formulae, $Z^1$ represents an oxygen atom or a sulfur atom. When a plurality of $Z^1$ are present, they may be the same or different.

[Chemical Formula 5]

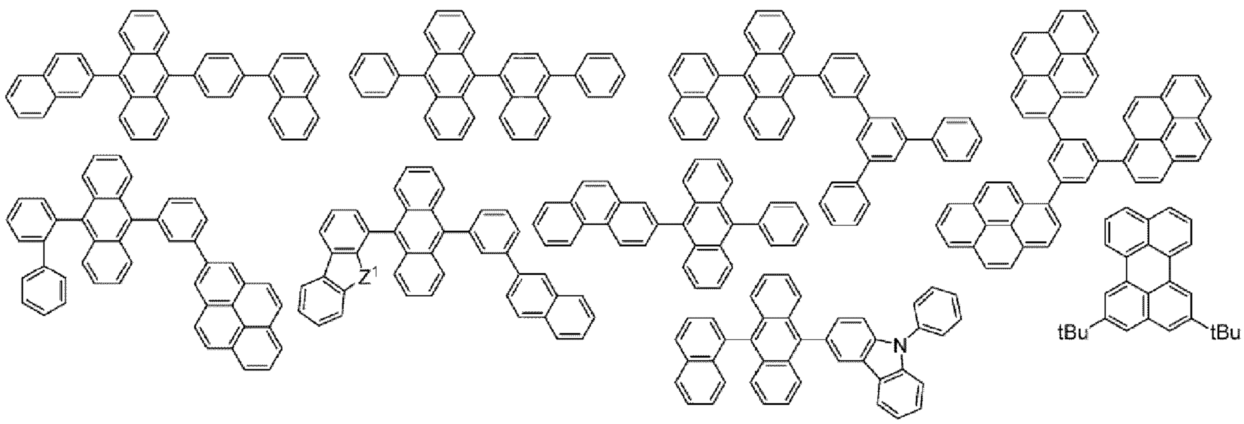

[Chemical Formula 6]

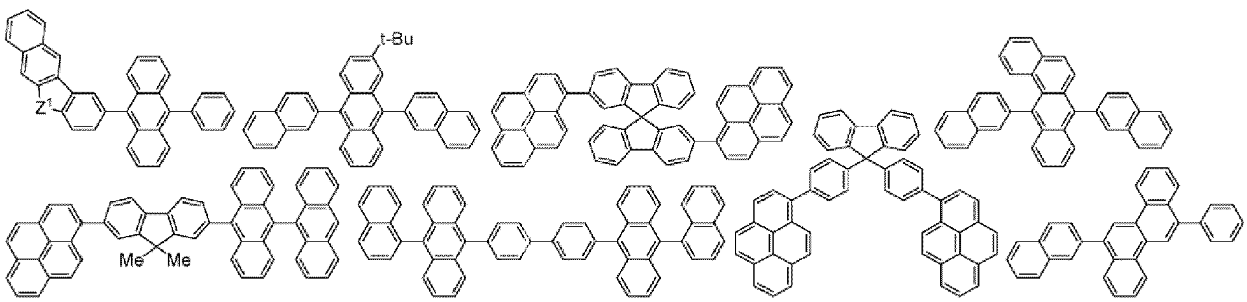

(Polymer host material)

The polystyrene-equivalent number-average molecular weight of the polymer host material is preferably $5\times10^3$ to $1x10^6$, more preferably $1\times10^4$ to $5\times10^5$, and further preferably $2\times10^4$ to $2x10^5$. The polystyrene-equivalent weight-average molecular weight of the polymer host material is preferably $1\times10^4$ to $2\times10^6$ , more preferably $2\times10^4$ to $1x10^6$, and further preferably $5\times10^5$ to $5\times10^5$.

The polymer host material may be any of a block copolymer, a random copolymer, an alternative copolymer and a graft copolymer, and may be in any form, however, copolymers obtained by copolymerizing multiple types of raw material monomers are preferable.

It is preferable that the polymer host material contains a condensed ring skeleton in which only three or more benzene rings are condensed, as a group obtained by removing from an aromatic hydrocarbon having a condensed ring skeleton in which only three or more benzene rings are condensed one or more hydrogen atoms bonding directly to carbon atoms constituting the condensed ring (condensed ring-containing aromatic hydrocarbon group), and this group optionally

has a substituent.

Since the initial deterioration of the light emitting device of the present embodiment is more suppressed, the polymer host material preferably contains a condensed ring-containing aromatic hydrocarbon group in the polymer compound, and more preferably contains a group obtained by removing from an aromatic hydrocarbon having a condensed ring skeleton in which only three or more benzene rings are condensed two hydrogen atoms bonding directly to carbon atoms constituting the condensed ring (divalent condensed ring-containing aromatic hydrocarbon group) in the polymer compound. This group optionally has a substituent.

In the polymer host material, the condensed ring-containing aromatic hydrocarbon group is preferably a group obtained by removing from a compound represented by the formula (FH) one or more (preferably 5 or less, more preferably 1 to 3, and further preferably 2) hydrogen atoms, since the initial deterioration of the light emitting device of the present embodiment is more suppressed.

In the polymer host material, the content of the condensed ring-containing aromatic hydrocarbon group contained in the polymer compound is usually 0.1% by mol to 100% by mol with respect to the total content of all constitutional units contained in the polymer compound, and it is preferably 1% by mol to 100% by mol, more preferably 10% by mol to 100% by mol and further preferably 30% by mol to 100% by mol since the initial deterioration of the light emitting device of the present embodiment is more suppressed.

The polymer host material may contain only one type of the condensed ring-containing aromatic hydrocarbon group or may contain two or more types thereof, and the number of the types is preferably 1 to 5, more preferably 1 to 3 and further preferably 1 since synthesis of the polymer host material is easy.

In the polymer host material, the examples and the preferable range of the substituent which the condensed ring-containing aromatic hydrocarbon group optionally has are the same as the examples and the preferable range of the substituent which the condensed ring-containing aromatic hydrocarbon optionally has.

The polymer host material may contain a constitutional unit other than the condensed ring-containing aromatic hydrocarbon group in the polymer compound, and preferably contains a constitutional unit other than the condensed ring-containing aromatic hydrocarbon group in the main chain of the polymer compound.

The constitutional unit other than the condensed ring-containing aromatic hydrocarbon group includes, for example, groups obtained by removing from an aromatic hydrocarbon group (preferably, an arylene group) other than the condensed ring-containing aromatic hydrocarbon group, a heterocyclic group (preferably, a divalent heterocyclic group) and an aromatic amine compound one or more hydrogen atoms (preferably, groups obtained by removing two hydrogen atoms), and these groups optionally have a substituent. The examples and the preferable range of this substituent are the same as the examples and the preferable range of the substituent which the condensed ring-containing aromatic hydrocarbon optionally has.

In the polymer host material, the total content of groups obtained by removing from the condensed ring-containing aromatic hydrocarbon group, an aromatic hydrocarbon group other than the condensed ring-containing aromatic hydrocarbon group, a heterocyclic group and an aromatic amine compound contained in the polymer compound one or more hydrogen atoms is usually 1% by mol to 100% by mol with respect to the total content of all constitutional units contained in the polymer compound, and it is preferably 50% by mol to 100% by mol and more preferably 70% by mol to 100% by mol since the initial deterioration of the light emitting device of the present embodiment is more suppressed.

The polymer host material may contain only one type of the constitutional unit other than the condensed ring-containing aromatic hydrocarbon group or may contain two or more types thereof in the polymer compound.

<Guest material>

In general, the aromatic amine compound means a compound containing a skeleton obtained by substitution with at least one of an amino group and a substituted amino group on an aromatic hydrocarbon group or an aromatic heterocyclic group (hereinafter, referred to also as "aromatic amine skeleton"). The aromatic amine skeleton is preferably a skeleton obtained by substitution with one or more substituted amino groups on an aromatic hydrocarbon group or an aromatic heterocyclic group, and more preferably a skeleton obtained by substitution with one or more substituted amino groups on an aromatic hydrocarbon group, since the initial deterioration of the light emitting device of the present embodiment is more suppressed.

The examples and the preferable range of the substituted amino group in the aromatic amine compound are the same as the examples and the preferable range of the substituted amino group as the substituent which the condensed ring-containing aromatic hydrocarbon optionally has.

The aromatic amine compound may contain only one type of the substituted amino group or may contain two or more types thereof, in the compound. Further, the aromatic amine compound may contain only either one of an amino group and a substituted amino group, or may contain only one of the each group or may contain two or more of the each group, in the compound.

The aromatic amine compound may contain only one type of the aromatic amine skeleton or may contain two or

more types thereof, in the compound. Further, the aromatic amine compound may contain only one aromatic amine skeleton, or may contain two or more of the skeleton, in the compound.

In the aromatic amine skeleton, the number of the amino group substituted on the aromatic hydrocarbon group or the aromatic heterocyclic group is usually 0 to 10, preferably 0 to 5, more preferably 0 to 3, and further preferably 0.

In the aromatic amine skeleton, the number of the substituted amino group substituted on the aromatic hydrocarbon group or the aromatic heterocyclic group is usually 1 to 10, and it is preferably 1 to 7, more preferably 1 to 5 and further preferably 1 to 3 since the initial deterioration of the light emitting device of the present embodiment is more suppressed.

In the aromatic amine skeleton, the total number of the amino group and the substituted amino group substituted on the aromatic hydrocarbon group or the aromatic heterocyclic group is usually 1 to 10, and it is preferably 1 to 7, more preferably 1 to 5 and further preferably 1 to 3 since the initial deterioration of the light emitting device of the present embodiment is more suppressed.

In the aromatic amine compound, the aromatic hydrocarbon group is preferably a group obtained by removing from a monocyclic or bicyclic to hexacyclic aromatic hydrocarbon one or more hydrogen atoms bonding directly to carbon atoms constituting the ring, more preferably a group obtained by removing from a tricyclic to pentacyclic aromatic hydrocarbon one or more hydrogen atoms bonding directly to carbon atoms constituting the ring, further preferably a group obtained by removing from anthracene, phenanthrene, fluorene, benzoanthracene, benzophenanthrene, benzofluorene or pyrene one or more hydrogen atoms bonding directly to carbon atoms constituting the ring, and particularly preferably a group obtained by removing from benzoanthracene, benzophenanthrene or pyrene one or more hydrogen atoms bonding directly to carbon atoms constituting the ring, and these groups optionally have a substituent, since the initial deterioration of the light emitting device of the present embodiment is more suppressed.

In the aromatic amine compound, the aromatic heterocyclic group includes, for example, groups obtained by removing from a monocyclic or bicyclic to heptacyclic aromatic heterocyclic compound among heterocyclic compounds exemplified in the section of the heterocyclic group described above one or more hydrogen atoms bonding directly to atoms constituting the ring, and is preferably a group obtained by removing from a monocyclic or bicyclic to hexacyclic aromatic heterocyclic compound one or more hydrogen atoms bonding directly to atoms constituting the ring, more preferably a group obtained by removing from a tricyclic to pentacyclic aromatic heterocyclic compound one or more hydrogen atoms bonding directly to atoms constituting the ring, and further preferably a group obtained by removing from dibenzofuran, dibenzothiophene, carbazole, azaanthracene, diazaanthracene, azaphenanthrene, diazaphenanthrene, benzocarbazole, benzonaphthofuran or benzonaphthothiophene one or more hydrogen atoms bonding directly to atoms constituting the ring, and these groups optionally have a substituent.

In the aromatic amine compound, the substituent which the aromatic hydrocarbon group and the aromatic heterocyclic group optionally have is a substituent other than the amino group and the substituted amino group, and is preferably a halogen atom, an alkyl group, a cycloalkyl group, an alkoxy group, a cycloalkoxy group, an aryl group or a monovalent heterocyclic group, more preferably an alkyl group, a cycloalkyl group, an aryl group or a monovalent heterocyclic group, and further preferably an alkyl group or a cycloalkyl group, and these groups optionally further have a substituent.

In the aromatic amine compound, the examples and the preferable range of the aryl group and the monovalent heterocyclic group as the substituent which the aromatic hydrocarbon group and the aromatic heterocyclic group optionally have are the same as the examples and the preferable range of the aryl group and the monovalent heterocyclic group as the substituent which the aromatic hydrocarbon in which only three or more benzene rings are condensed optionally has, respectively.

In the aromatic amine compound, the examples and the preferable range of the substituent which the substituent which the aromatic hydrocarbon group and the aromatic heterocyclic group optionally have optionally further has are the same as the examples and the preferable range of the substituent which the substituent which the aromatic hydrocarbon in which only three or more benzene rings are condensed optionally has optionally further has.

The guest material may further contain a compound other than the aromatic amine compound, however, it is preferable that the aromatic amine compound is contained as the main component since the initial deterioration of the light emitting device of the present embodiment is more suppressed. The content ratio of the aromatic amine compound occupying the guest material may be, for example, 10% by mass or more, and it is preferably 30% by mass or more, more preferably 50% by mass or more, further preferably 70% by mass or more, particularly preferably 90% by mass or more and especially preferably 95% by mass or more and may also be 100% by mass since the initial deterioration of the light emitting device of the present embodiment is more suppressed.

The guest material may contain only one type of the aromatic amine compound or may contain two or more types thereof. In the claimed invention, the guest material specifically contains at least an aromatic amine compound that is a compound represented by the formula (FB). When the guest material further contains a compound other than the aromatic amine compound, the guest material may contain only one type of the compound other than the aromatic amine compound or may contain two or more types thereof.

In general, an aromatic amine compound may be a polymer compound (hereinafter, referred to also as "polymer guest material") or a low-molecular compound (hereinafter, referred to also as "low-molecular guest material"), with the low-molecular guest material being preferred.

(Low-molecular guest material)

The molecular weight of the low-molecular guest material is usually $1\times10^2$ to $1\times10^4$, preferably $2\times10^2$ to $5\times10^3$, more preferably $3\times10^2$ to $2\times10^3$, and further preferably $4\times10^2$ to $1\times10^3$.

The total number of the amino groups and the substituted amino groups contained in the low-molecular guest material is usually 1 to 20, and it is preferably 1 to 15, more preferably 1 to 10, further preferably 1 to 5 and particularly preferably 1 to 3 since the initial deterioration of the light emitting device of the present embodiment is more suppressed.

The total number of the amino groups contained in the low-molecular guest material is usually 0 to 10, preferably 0 to 5, more preferably 0 to 3, and further preferably 0.

The total number of the substituted amino groups contained in the low-molecular guest material is usually 1 to 20, and it is preferably 1 to 15, more preferably 1 to 10, further preferably 1 to 5 and particularly preferably 1 to 3 since the initial deterioration of the light emitting device of the present embodiment is more suppressed.

The low-molecular guest material may contain only one type of the substituted amino group or may contain two or more types thereof, and the number of the types is preferably 1 to 10, more preferably 1 to 5, further preferably 1 to 3 and particularly preferably 1 since synthesis of the low-molecular host material is easy.

The total number of the aromatic amine skeletons contained in the low-molecular guest material is usually 1 to 10, and it is preferably 1 to 7, more preferably 1 to 5, further preferably 1 to 3 and particularly preferably 1 since the initial deterioration of the light emitting device of the present embodiment is more suppressed.

The low-molecular guest material may contain only one type of the aromatic amine skeleton or may contain two or more types thereof, and the number of the types is preferably 1 to 5, more preferably 1 to 3 and further preferably 1 since synthesis of the low-molecular host material is easy.

[Compound represented by the formula (FB)]

In the claimed invention, the guest material contains at least a low-molecular guest material that is a compound represented by the formula (FB), since the initial deterioration of the light emitting device of the present embodiment is more suppressed.

$n^{1B}$ is usually an integer of 1 or more and 10 or less, and it is preferably an integer of 1 or more and 7 or less, more preferably an integer of 1 or more and 5 or less and further preferably an integer of 1 or more and 3 or less since synthesis of a compound represented by the formula (FB) is easy.

$Ar^{1B}$ is preferably an aromatic hydrocarbon group optionally having a substituent, since the initial deterioration of the light emitting device of the present embodiment is more suppressed.

The examples and the preferable range of the aromatic hydrocarbon group and the aromatic heterocyclic group represented by $Ar^{1B}$ are the same as the examples and the preferable range of the aromatic hydrocarbon group or the aromatic heterocyclic group in the aromatic amine compound, respectively.

The examples and the preferable range of the substituent which $Ar^{1B}$ optionally has are the same as the examples and the preferable range of the substituent which the aromatic hydrocarbon group and the aromatic heterocyclic group optionally have in the aromatic amine compound.

$R^{1B}$ is preferably a substituted amino group, and this group optionally further has a substituent, since the initial deterioration of the light emitting device of the present embodiment is more suppressed.

The examples and the preferable range of the substituted amino group represented by $R^{1B}$ are the same as the examples and the preferable range of the substituted amino group in the aromatic amine compound.

The examples and the preferable range of the substituent which $R^{1B}$ optionally has are the same as the examples and the preferable range of the substituent which the substituent which the aromatic hydrocarbon group and the aromatic heterocyclic group optionally have optionally further has in the aromatic amine compound.

As the low-molecular guest material, compounds represented by the following formulae and compounds described in examples are exemplified. These compounds optionally have a substituent. In the formulae, $Z^1$ represents the same meaning as described above.

[Chemical Formula 7]

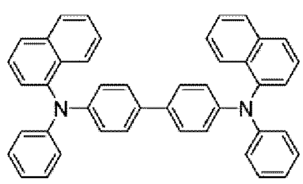
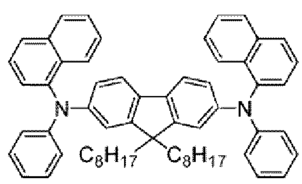
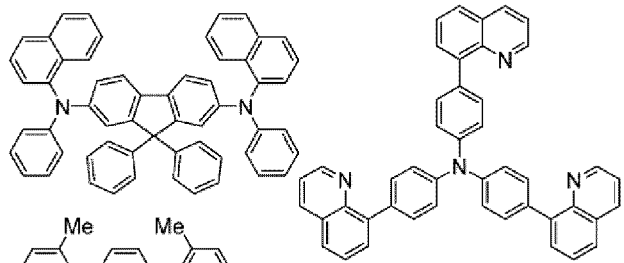
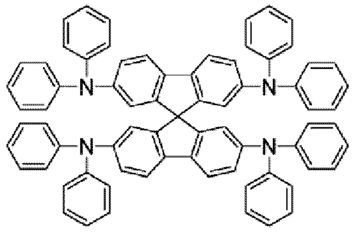
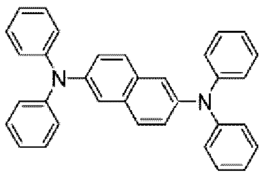
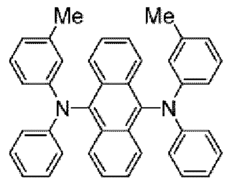
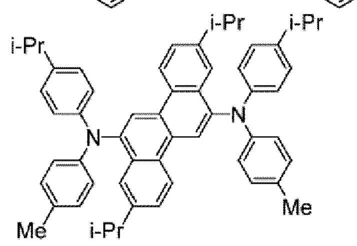

[Chemical Formula 8]

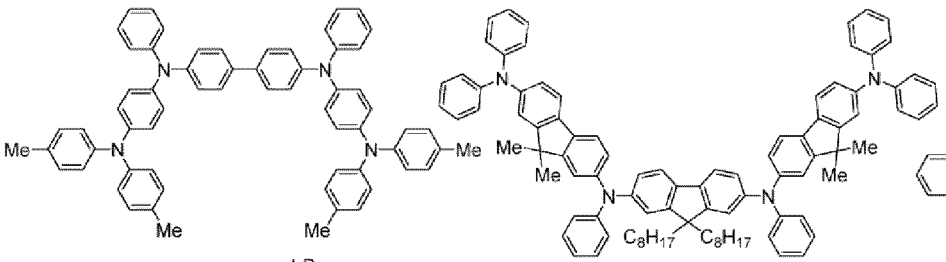
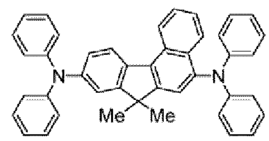
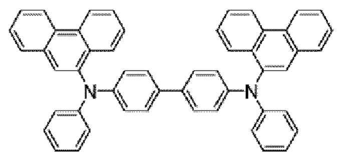
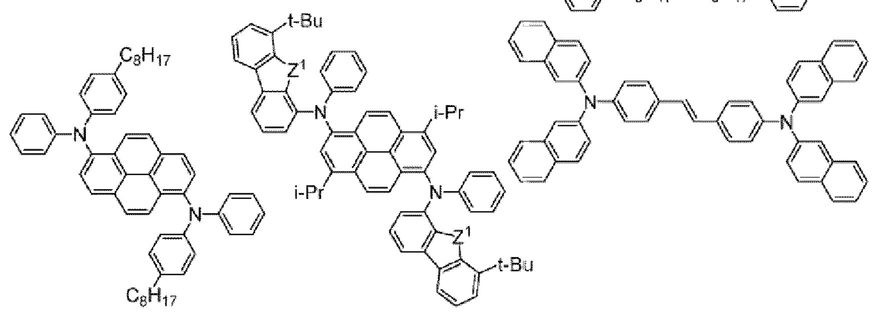
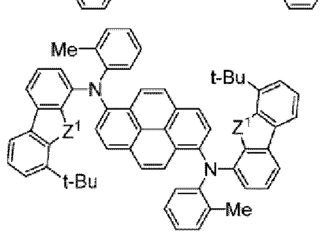

(Polymer guest material)

The preferable ranges of the polystyrene-equivalent number-average molecular weight and weight-average molecular weight of the polymer guest material are the same as the preferable ranges of the polystyrene-equivalent number-average molecular weight and weight-average molecular weight of the polymer host material, respectively.

The polymer guest material may be any of a block copolymer, a random copolymer, an alternative copolymer and a graft copolymer, and may also be in other form, however, copolymers obtained by copolymerizing multiple types of raw material monomers are preferable.

The polymer guest material can be said to be a polymer compound containing a constitutional unit composed of an aromatic amine skeleton. The polymer guest material preferably contains an aromatic amine skeleton in the main chain of the polymer compound, since the initial deterioration of the light emitting device of the present embodiment is more suppressed.

In the polymer guest material, the aromatic amine skeleton is preferably a group obtained by removing from a compound represented by the formula (FB) one or more (preferably 5 or less, more preferably 1 to 3, and further preferably 2) hydrogen atoms, since the initial deterioration of the light emitting device of the present embodiment is more suppressed.

In the polymer guest material, the content of the aromatic amine skeleton contained in the polymer compound is usually 0.1% by mol to 100% by mol, with respect to the total content of all constitutional units contained in the polymer compound, and it is preferably 1% by mol to 100% by mol, more preferably 5% by mol to 100% by mol and further preferably

10% by mol to 100% by mol since the initial deterioration of the light emitting device of the present embodiment is more suppressed.

The polymer guest material may contain only one type of the aromatic amine skeleton or two or more types thereof in the polymer compound, and the number of the types is preferably 1 to 5, more preferably 1 to 3 and further preferably 1 since synthesis of the polymer guest material is easy.

In the polymer guest material, the examples and the preferable range of the substituent which the aromatic amine skeleton optionally has are the same as the examples and the preferable range of the substituent which the aromatic hydrocarbon group and the aromatic heterocyclic group optionally have in the aromatic amine compound.

The polymer guest material may contain a constitutional unit other than the aromatic amine skeleton in the polymer compound, and preferably contains a constitutional unit other than the aromatic amine skeleton in the main chain of the polymer compound.

The constitutional unit other than the aromatic amine skeleton includes, for example, aromatic hydrocarbon groups (preferably, an arylene group) and heterocyclic groups (preferably, a divalent heterocyclic group), and these groups optionally have a substituent. The examples and the preferable range of this substituent are the same as the examples and the preferable range of the substituent which the condensed ring-containing aromatic hydrocarbon optionally has.

In the polymer guest material, the total content of the aromatic amine skeleton, the aromatic hydrocarbon group and the heterocyclic group contained in the polymer compound is usually 1% by mol to 100% by mol, with respect to the total content of all constitutional units contained in the polymer compound, and it is preferably 50% by mol to 100% by mol and more preferably 70% by mol to 100% by mol since the initial deterioration of the light emitting device of the present embodiment is more suppressed.

The polymer guest material may contain only one type of the constitutional unit other than the aromatic amine skeleton or may contain two or more types thereof, in the polymer compound.

<Amount ($C^1$) of sodium atom contained in guest material>

In the composition for light emitting device of the present embodiment, the amount ($C^1$) of sodium atoms contained in the guest material is usually 40000 ppb by mass or less with respect to the total amount of the guest material. The phrase "the amount of sodium atoms contained in the guest material" is not intended that the guest material contains sodium atoms, and the guest material may or may not contain sodium atoms. In the guest material of the present embodiment, the amount of sodium atoms is preferably 4000 ppb by mass or less, more preferably 400 ppb by mass or less, further preferably 380 ppb by mass or less, particularly preferably 150 ppb by mass or less, especially preferably 50 ppb by mass or less, especially more preferably 10 ppb by mass or less, especially further preferably 1 ppb by mass or less and especially particularly preferably 0 ppb by mass since the initial deterioration of the light emitting device of the present embodiment is more suppressed.

Regarding the amount ($C^1$) of sodium atoms contained in the guest material of the present embodiment, when one type of the guest material of the present embodiment is present, the amount of sodium atoms in that one type of the guest material is $C^1$, and when the guest material of the present embodiment is composed of a plurality of types of compounds having different amounts of sodium atoms, C1 is calculated according to the amounts of sodium atoms in the plurality of types of compounds and the mass ratio of each compound. The specific method for calculating $C^1$ is explained using Example D1 and Example D2 described later.

First, in Example D1, since the amount of sodium atoms in a compound EM2 measured by an ICP/MS method is not higher than the detection limit, $C^1$ is 0 ppb by mass.

Next, in Example D2, the amounts of sodium atoms in a compound EM1 and a compound EM2 measured by an ICP/MS method are 420 ppb by mass and not higher than the detection limit (namely, 0 ppb by mass), respectively. The mass ratio of the compound EM1 to the compound EM2 is compound EM1:compound EM2 = 1:9.

Hence, $C^1$ in Example D2 can be determined from the amounts of sodium atoms contained in the compound EM1 and the compound EM2 and the charge amounts thereof, and calculated as described below.

$$C^1 = \{420\times1/(1+9)\}+\{0\times9/(1+9)\} = 42.0 \text{ ppb by mass}$$

Similarly, $C^1$ in Example D3 is 0 ppb by mass.

<Amount ($C^H$) of sodium atom contained in host material>

In the composition for light emitting device of the present embodiment, the amount ($C^H$) of sodium atoms

contained in the host material is usually 41000 ppb by mass or less with respect to the total amount of the host material. The phrase "the amount of sodium atoms contained in the host material" is not intended that the host material contains sodium atoms, and the host material may or may not contain sodium atoms. In the host material of the present embodiment, the amount of sodium atoms is preferably 4100 ppb by mass or less, more preferably 410 ppb by mass or less, further preferably 100 ppb by mass or less, particularly preferably 50 ppb by mass or less, especially preferably 10 ppb by mass or less, especially more preferably 5 ppb by mass or less, especially further preferably 1 ppb by mass or less and especially particularly preferably 0 ppb by mass since the initial deterioration of the light emitting device of the present embodiment is more suppressed.

$C^H$ can be determined in the same manner as the specific method for calculating $C^1$ described above.

For example, in Example D1, $C^H$ is 0 ppb by mass. In Example D2, $C^H$ is 0 ppb by mass. In Example D3, $C^H$ is 9.1 ppb by mass.

<$C^1$ and $C^H$ reduction method>

The method of reducing $C^1$ and $C^H$ includes, for example, purification.

The purification includes known purification methods described in 4th Edition Experimental Chemistry Course (1993, Maruzen), 5th Edition Experimental Chemistry Course (2007, Maruzen), New Experimental Chemistry Course (1975, Maruzen), Guide to Organic Chemical Experiment (1988, Kagaku-Dojin Publishing Company, Inc.), and the like.

The purification includes, for example, sublimation, extraction, reprecipitation, recrystallization, chromatography and adsorption.

The purification method for the low-molecular guest material and the low-molecular host material is preferably sublimation, recrystallization, chromatography or adsorption, more preferably sublimation or recrystallization and further preferably sublimation, since the mount of sodium atoms can be more reduced.

The purification method for the polymer guest material and the polymer host material is preferably reprecipitation, chromatography or adsorption, since the mount of sodium atoms can be more reduced.

When the purification is performed twice or more, the methods may be the same or different.

In sublimation, the degree of vacuum and the sublimation temperature may be appropriately set according to the material to be sublimated. The degree of vacuum is preferably $1\times10^{-10}$ Pa to $1\times10^{5}$ Pa, more preferably $1\times10^{-7}$ Pa to $1\times10^{2}$ Pa, further preferably $1\times10^{-5}$ Pa to 1 Pa, and particularly preferably $1\times10^{-4}$ Pa to $1\times10^{-2}$ Pa. The sublimation temperature is preferably -100°C to 1000°C, more preferably 0°C to 700°C, further preferably 100°C to 500°C, and particularly preferably 200°C to 350°C.

The extraction is preferably liquid separation, or solid-liquid extraction with a Soxhlet extractor.

The solvent used for extraction includes, for example, alcohol solvents such as methanol, ethanol, propanol, ethylene glycol, glycerin, 2-methoxyethanol, 2-ethoxyethanol and the like; ether solvents such as diethyl ether, tetrahydrofuran (THF), dioxane , cyclopentyl methyl ether, diglyme and the like; halogen-based solvents such as methylene chloride, chloroform and the like; nitrile solvents such as acetonitrile, benzonitrile and the like; hydrocarbon solvents such as hexane, decalin, toluene, xylene, mesitylene and the like; amide solvents such as N,N-dimethylformamide, N,N-dimethylacetamide and the like; acetone, dimethyl sulfoxide, and water. The solvent may be used singly or in combination of two or more.

The chromatography is preferably column chromatography.

As the filler used in column chromatography, silica gel or alumina is preferable.

The examples of the solvent used for chromatography are the same as the examples of the solvent used for extraction.

The examples of the solvent used for reprecipitation and recrystallization are the same as the examples of the solvent used for extraction.

As the adsorption, a treatment with an adsorbent is preferred. The adsorbent is preferably activated carbon, silica gel, alumina or Celite.

The treatment with an adsorbent is usually performed in a solvent. The examples of the solvent used for the treatment with an adsorbent are the same as the examples of the solvent used for extraction.

<Composition for light emitting device>

The composition for light emitting device of the present embodiment contains a host material and a guest material.

The composition for light emitting device of the present embodiment may contain only one type or two or more types of each of the host material and the guest material.

In the composition for light emitting device of the present embodiment, the maximum peak wavelength of the emission spectrum of the host material at room temperature is preferably shorter than the maximum peak wavelength of

the emission spectrum of the guest material at room temperature.

In the composition for light emitting device of the present embodiment, the maximum peak wavelength of the emission spectrum of the host material at room temperature is preferably 300 nm or more and 500 nm or less, more preferably 330 nm or more and 480 nm or less, and further preferably 360 nm or more and 460 nm or less.

In the composition for light emitting device of the present embodiment, the maximum peak wavelength of the emission spectrum of the guest material at room temperature is preferably 380 nm or more and 500 nm or less, more preferably 400 nm or more and 490 nm or less, and further preferably 430 nm or more and 480 nm or less.

The maximum peak wavelength of the emission spectrum of the host material and the guest material can be evaluated by dissolving the measurement object in an organic solvent such as xylene, toluene, chloroform, tetrahydrofuran and the like to prepare a dilute solution ($1\times10^{-6}$% by mass to $1\times10^{-3}$% by mass) and measuring the PL spectrum of the dilute solution at room temperature. As the organic solvent for dissolving the measurement object, toluene or xylene is preferable.

In the composition for light emitting device of the present embodiment, the total amount of sodium atoms contained in the host material and sodium atoms contained in the guest material is 400 ppb by mass or less, with respect to the total amount of the host material and the guest material, and it is preferably 390 ppb by mass or less, more preferably 370 ppb by mass or less, further preferably 100 ppb by mass or less, particularly preferably 15 ppb by mass or less, especially preferably 4 ppb by mass or less, especially more preferably 2 ppb by mass or less, especially further preferably 1 ppb by mass or less and especially particularly preferably 0 ppb by mass since the initial deterioration of the light emitting device of the present embodiment is suppressed. In the composition for light emitting device of the present embodiment, the total amount of sodium atoms contained in the host material and sodium atoms contained in the guest material is preferably 0.01 ppb by mass or more, more preferably 0.1 ppb by mass or more, further preferably 1 ppb by mass or more, particularly preferably 2 ppb by mass or more, especially preferably 4 ppb by mass or more, especially more preferably 6 ppb by mass or more, especially further preferably 10 ppb by mass or more and especially particularly preferably 15 ppb by mass or more, with respect to the total amount of the host material and the guest material, since the initial deterioration of the light emitting device of the present embodiment can be controlled.

In the present embodiment, the reason why the initial deterioration of the light emitting device is suppressed is considered as follows.

The aromatic compound contained as the host material in the composition for light emitting device of the present embodiment has a condensed ring skeleton in which only three or more benzene rings are condensed. The present inventors consider that such a condensed ring skeleton electrically interacts with the aromatic amine compound contained in the guest compound. In contrast, the present inventors consider that the aromatic amine compound contained as the guest material in the composition for light emitting device of the present embodiment electrically interacts with the aromatic compound contained as the host material. Further, the present inventors consider that when the total amount of sodium atoms contained in the host material and sodium atoms contained in the guest material exceeds a predetermined amount in the composition for light emitting device of the present embodiment, sodium atoms exert an adverse effect on the interaction described above. Furthermore, it is considered that this adverse effect causes a lowering of the light emitting property, the charge transporting property or the charge injection property of the composition for light emitting device of the present embodiment or renders the charge balance of the light emitting device of the present embodiment to be disturbed, leading to the initial deterioration of the light emitting device of the present embodiment.

Thus, the present inventors consider based on the above idea that when the total amount of sodium atoms contained in the host material and sodium atoms contained in the guest material is within a specific range in the present embodiment, the above-described influence by sodium atoms is suppressed and the effect of suppression of the initial deterioration of the light emitting device is obtained.

The total amount (ppb by mass) of sodium atoms contained in the host material and sodium atoms contained in the guest material is represented by $C^HW^H+C^1W^1$, when the ratio of the mass of the host material to the total mass of the host material and the guest material is represented by $W^H$ and the ratio of the mass of the guest material to the total mass of the host material and the guest material is represented by $W^1$.

$W^H$ is usually 0.01 to 0.9999, and it is preferably 0.30 to 0.999, more preferably 0.50 to 0.995, further preferably 0.70 to 0.99 and particularly preferably 0.85 to 0.95 since the initial deterioration of the light emitting device of the present embodiment is more suppressed.

$W^1$ is usually 0.0001 to 0.99, and it is preferably 0.001 to 0.70, more preferably 0.005 to 0.50, further preferably 0.01 to 0.30 and particularly preferably 0.05 to 0.15 since the initial deterioration of the light emitting device of the present embodiment is more suppressed.

The specific method for calculating $W^H$ and $W^1$ is explained using Example D1 and Example D2 described later.

First, in Example D1, the mass ratio of a compound H2 (host material) to a compound EM2 (guest material) is compound H2:compound EM2 = 90:10.

Hence, $W^H$ and $W^1$ in Example D1 can be determined from the charge amounts, and calculated as follows.

$$W^H = 90/(90+10) = 0.90$$

$$W^1 = 10/(90+10) = 0.10$$

In Example D2, the mass ratio of a compound H2, a compound EM1 and a compound EM2 is compound H2:compound EM1:compound EM2 = 90:1:9.

Hence, $W^H$ and $W^1$ in Example D2 can be determined from the charge amounts, and calculated as follows.

$$W^H = 90/(90+1+9) = 0.90$$

$$W^1 = (1+9)/(90+1+9) = 0.10$$

In the same manner, $W^H$ and $W^1$ in Example D3 are calculated as follows.

$$W^H = (2+88)/(2+88+10) = 0.90$$

$$W^1 = 10/(2+88+10) = 0.10$$

$C^HW^H+C^1W^1$ can be calculated by calculating $C^1$, $C^H$, $W^1$ and $W^H$ as described above.

For example, $C^HW^H+C^1W^1$ in Example D1 is calculated as follows.

$$C^H W^H + C^1 W^1 = (0\times0.90)+(0\times0.10) = 0 \text{ ppb by mass}$$

For example, $C^HW^H+C^1W^1$ in Example D2 is calculated as follows.

$$C^H W^H + C^1 W^1 = (0\times0.90)+(42.0\times0.10) = 4.2 \text{ ppb by mass}$$

For example, $C^HW^H+C^1W^1$ in Example D3 is calculated as follows.

$$C^H W^H + C^1 W^1 = (9.1\times0.90)+(0\times0.10) = 8.2 \text{ ppb by mass}$$

$C^HW^H+C^1W^1$ is 400 ppb by mass or less, and it is preferably 390 ppb by mass or less, more preferably 370 ppb by mass or less, further preferably 100 ppb by mass or less, particularly preferably 15 ppb by mass or less, especially preferably 4 ppb by mass or less, especially more preferably 2 ppb by mass or less, especially further preferably 1 ppb by mass or less and especially particularly preferably 0 ppb by mass since the initial deterioration of the light emitting device of the present embodiment is suppressed. In contrast, $C^HW^H+C^1W^1$ is preferably 0.01 ppb by mass or more, more preferably 0.1 ppb by mass or more, further preferably 1 ppb by mass or more, particularly preferably 2 ppb by mass or more, especially preferably 4 ppb by mass or more, especially more preferably 6 ppb by mass or more, especially further preferably 10 ppb by mass or more and especially particularly preferably 15 ppb by mass or more since the initial deterioration of the light emitting device of the present embodiment can be controlled.

(Other components)

The composition for light emitting device of the present embodiment may be a composition containing a host material, a guest material, and at least one material selected from the group consisting of a hole transporting material, a hole injection material, an electron transporting material, an electron injection material, a light emitting material, an antioxidant and a solvent. However, the hole transporting material, the hole injection material, the electron transporting material, the electron injection material and the light emitting material are different from the host material and the guest material.

When the composition for light emitting device of the present embodiment further contains at least one material selected from the group consisting of a hole transporting material, a hole injection material, an electron transporting material, an electron injection material, a light emitting material, an antioxidant and a solvent, it is preferable that the amount of sodium atoms contained in them is reduced by the above-described purification.

[Ink]

A composition containing a host material, a guest material and a solvent (hereinafter, referred to as "ink") is suitable for fabrication of a light emitting device using a wet method such as, for example, a spin coat method, a casting method, a microgravure coat method, a gravure coat method, a bar coat method, a roll coat method, a wire bar coat method, a dip coat method, a spray coat method, a screen printing method, a flexo printing method, an offset printing method, an inkjet printing method, a capillary coat method, a nozzle coat method and the like. The viscosity of the ink may be adjusted according to the type of the printing method, and it is preferably 1 mPa•s to 20 mPa•s at 25°C.

The solvent contained in the ink is preferably a solvent capable of dissolving or uniformly dispersing the solid content in the ink. The solvent includes, for example, chlorine-based solvents, ether solvents, aromatic hydrocarbon solvents, aliphatic hydrocarbon solvents, ketone solvents, ester solvents, polyhydric alcohol solvents, alcohol solvents, sulfoxide solvents, and amide solvents.

In the ink, the blending amount of the solvent is usually 1000 parts by mass to 100000 parts by mass, when the sum of the host material and the guest material is taken as 100 parts by mass.

The solvent may be used singly or in combination of two or more.

[Hole transporting material]

The hole transporting material is classified into a low-molecular compound and a polymer compound, and is preferably a polymer compound having a crosslinkable group.

The polymer compound includes, for example, polyvinylcarbazole and derivatives thereof; and polyarylenes having an aromatic amine structure in the side chain or main chain, and derivatives thereof. The polymer compound may be a compound to which an electron accepting site such as fullerene, tetrafluorotetracyanoquinodimethane, tetracyanoethylene and trinitrofluorene and the like is bonded.

In the composition for light emitting device of the present embodiment, when a hole transporting material is contained, the blending amount of the hole transporting material is usually 1 part by mass to 400 parts by mass, when the sum of the host material and the guest material is taken as 100 parts by mass.

The hole transporting material may be used singly or in combination of two or more.

[Electron transporting material]

The electron transporting material is classified into a low-molecular compound and a polymer compound. The electron transporting material may have a crosslinkable group.

The low-molecular compound includes, for example, a metal complex having 8-hydroxyquinoline as a ligand; oxadiazole, anthraquinodimethane, benzoquinone, naphthoquinone, anthraquinone, tetracyanoanthraquinodimethane, fluorenone, diphenyldicyanoethylene and diphenoquinone, and derivatives thereof.

The polymer compound includes, for example, polyphenylene, polyfluorene, and derivatives thereof. The polymer compound may be doped with a metal.

In the composition for light emitting device of the present embodiment, when an electron transporting material is contained, the blending amount of the electron transporting material is usually 1 part by mass to 400 parts by mass, when the sum of the host material and the guest material is taken as 100 parts by mass.

The electron transporting material may be used singly or in combination of two or more.

[Hole injection material and electron injection material]

The hole injection material and the electron injection material are each classified into a low-molecular compound and a polymer compound. The hole injection material and the electron injection material may have a crosslinkable group.

The low-molecular compound includes, for example, metal phthalocyanines such as copper phthalocyanine and the like; carbon; oxides of metals such as molybdenum, tungsten and the like; and metal fluorides such as lithium fluoride, cesium fluoride, potassium fluoride and the like.

The polymer compound includes, for example, conductive polymers such as polyaniline, polythiophene, polypyrrole, polyphenylenevinylene, polythienylenevinylene, polyquinoline and polyquinoxaline, and derivatives thereof; and polymers containing an aromatic amine structure in the main chain or side chain, and the like.

In the composition for light emitting device pf the present embodiment, when a hole injection material and/or an electron injection material is contained, the blending amount of each of the hole injection material and the electron injection material is usually 1 part by mass to 400 parts by mass, when the sum of the host material and the guest material is taken as 100 parts by mass.

The hole injection material and the electron injection material may each be used singly or in combination of two or

more.

(Ion doping)

When the hole injection material or the electron injection material contains a conductive polymer, the electrical conductivity of the conductive polymer is preferably $1 \times 10^{-5}$ S/cm to $1 \times 10^{3}$ S/cm. In order to set the electric conductivity of the conductive polymer within the above range, the conductive polymer can be doped with an appropriate amount of ions. The types of ions for doping are anions for hole injection materials and cations for electron injection materials. The anion includes, for example, a polystyrenesulfonate ion, an alkylbenzenesulfonate ion and a camphorsulfonate ion. The cation includes, for example, a lithium ion, a potassium ion, and a tetrabutylammonium ion.

The ions for doping may be used singly or in combination of two or more.

[Light emitting material]

The light emitting material is classified into a low-molecular compound and a polymer compound. The light emitting material may have a crosslinkable group.

The low-molecular compound includes, for example, naphthalene and derivatives thereof, anthracene and derivatives thereof, perylene and derivatives thereof, and triplet light emitting complexes having iridium, platinum or europium as a central metal.

The polymer compound includes, for example, arylene groups such as a phenylene group, a naphthalenediyl group, a fluorenediyl group, a phenanthrenediyl group, a dihydrophenanthrenediyl group, an anthracenediyl group and a pyrenediyl group and the like; aromatic amine residues such as a group obtained by removing from an aromatic amine two hydrogen atoms, and the like; and polymer compounds containing a divalent heterocyclic group such as a carbazolediyl group, a phenoxazinediyl group and a phenothiazinediyl group and the like.

In the composition for light emitting device of the present embodiment, when a light emitting material is contained, the content of the light emitting material is usually 0.1 parts by mass to 400 parts by mass, when the sum of the host material and the guest material is taken as 100 parts by mass.

The light emitting material may be used singly or in combination of two or more.

[Antioxidant]

The antioxidant may be a compound that is soluble in the same solvent as for the host material and the guest material and does not inhibit light emission and charge transportation, and includes, for example, a phenol-based antioxidant and a phosphorus-based antioxidant.

In the composition for light emitting device of the present embodiment, when an antioxidant is contained, the blending amount of the antioxidant is usually 0.001 parts by mass to 10 parts by mass, when the sum of the host material and the guest material is taken as 100 parts by mass.

The antioxidant may be used singly or in combination of two or more.

<Film>

The film contains the composition for light emitting device of the present embodiment, and is suitable as a light emitting layer in the light emitting device. The film can be fabricated, for example, by a wet method using an ink. Further, the film can be fabricated, for example, by a dry method such as a vacuum vapor deposition method and the like. The method for fabricating the film by a dry method includes, for example, a method of vapor-depositing the composition for light emitting device of the present embodiment, and a method of co-vapor-depositing the host material and the guest material.

The thickness of the film is, usually 1 nm to 10 μm.

<Light emitting device>

The light emitting device of the present embodiment contains the composition for light emitting device described above.

The constitution of the light emitting device of the present embodiment has, for example, an anode, a cathode, and an organic layer containing the composition for light emitting device of the present embodiment disposed between the above-described anode and the above-described cathode.

[Layer constitution]

The layer containing the composition for light emitting device of the present embodiment is usually one or more selected from the group consisting of a light emitting layer, a hole transporting layer, a hole injection layer, and electron transporting layer and an electron injection layer, and preferable is a light emitting layer. These layers contain a light emitting material, a hole transporting material, a hole injection material, an electron transporting material and an electron injection material, respectively. These layers can be formed from a light emitting material, a hole transporting material, a hole injection material, an electron transporting material and an electron injection material, respectively, using the same method as that for film fabrication described above.
The light emitting device has a light emitting layer between an anode and a cathode. The light emitting device of the present embodiment preferably has at least one of a hole injection layer and a hole transporting layer between an anode and a light emitting layer from the standpoint of hole injectability and hole transportability, and preferably has at least one of an electron injection layer and an electron transporting layer between a cathode and a light emitting layer from the standpoint of electron injectability and electron transportability.
The materials of the hole transporting layer, the electron transporting layer, the light emitting layer, the hole injection layer and the electron injection layer include the hole transporting material, the electron transporting material, the light emitting material, the hole injection material and the electron injection material described above, respectively, in addition to the composition for light emitting device of the present embodiment.
When the material of the hole transporting layer, the material of the electron transporting layer and the material of the light emitting layer are soluble in a solvent used in forming a layer adjacent to the hole transporting layer, the electron transporting layer and the light emitting layer, respectively, it is preferable that the material has a crosslinkable group for preventing the material from being dissolved in the solvent. After forming each layer using the material having a crosslinkable group, the layer can be insolubilized by crosslinking the crosslinkable group.
The method of forming each of the light emitting layer, the hole transporting layer, the electron transporting layer, the hole injection layer, the electron injection layer and the like in the light emitting device of the present embodiment includes, for example, dry methods such as a method of vacuum vapor deposition from a powder and the like and wet methods such as a method by film formation from a solution or melted state and the like when a low molecular compound is used, and includes, for example, wet methods such as a method by film formation from a solution or melted state and the like when a polymer compound is used. The order, the number and the thickness of the layers to be laminated are adjusted in consideration of, for example, the light emission efficiency and the initial deterioration.

[Substrate/electrode]

The substrate in the light emitting device may be a substrate on which an electrode can be formed and which does not chemically change when forming an organic layer, and is a substrate made of a material such as, for example, glass, plastic, silicon and the like. In the case of an opaque substrate, it is preferable that the electrode farthest from the substrate is transparent or semitransparent.
The material of the anode includes, for example, conductive metal oxides and semitransparent metals, preferably, indium oxide, zinc oxide, tin oxide; conductive compounds such as indium•tin•oxide (ITO), indium•zinc•oxide, and the like; a composite of argentine, palladium and copper (APC); NESA, gold, platinum, silver, and copper.
The material of the cathode includes, for example, metals such as lithium, sodium, potassium, rubidium, cesium, beryllium, magnesium, calcium, strontium, barium, aluminum, zinc, indium and the like; alloys composed of two or more of them; alloys composed of one or more of them and one or more of silver, copper, manganese, titanium, cobalt, nickel, tungsten and tin; and graphite and graphite intercalation compounds. The alloy includes, for example, a magnesium-silver alloy, a magnesium-indium alloy, a magnesium-aluminum alloy, an indium-silver alloy, a lithium-aluminum alloy, a lithium-magnesium alloy, a lithium-indium alloy, and a calcium-aluminum alloy.
The anode and the cathode each may take a laminated structure composed of two or more layers.
The light emitting device of the present embodiment can be suitably used as a light source for backlight of liquid crystal display devices, a light source for illumination devices, an organic EL lighting device, and, a display device of computers, televisions, mobile terminals and the like (for example, organic EL displays and organic EL televisions).
A suitable embodiment of the present invention is described above, but the present invention is not limited to the above-described embodiment.
For example, one aspect of the present invention may relate to a method for producing a composition for light emitting device containing a host material and a guest material blended therein.

<Production method (1)>

In one embodiment, the method for producing a composition for light emitting device may be a production method

of a composition for light emitting device comprising a host material preparation step of preparing a host material containing a condensed ring-containing aromatic compound, a guest material preparation step of preparing a guest material containing an aromatic amine compound, and a production step of mixing the host material and the guest material at a blending ratio such that the total amount of sodium atoms contained in the host material and sodium atoms contained in the guest material is 400 ppb by mass or less to obtain a composition for light emitting device (hereinafter, referred to also as "production method (1)").

In the production method (1), the host material preparation step may comprise a step (A-1) of preparing a condensed ring-containing aromatic compound containing sodium atoms mixed therein, and a step (A-2) of purifying at least a part of the condensed ring-containing aromatic compound prepared in the step (A-1) to remove at least a part of sodium atoms.

The content of sodium atoms in the condensed ring-containing aromatic compound prepared in the step (A-1) is not particularly restricted, and for example, may be 410 ppb by mass or more, may be 500 ppb by mass or more, may be 1000 ppb by mass or more, may be 5000 ppb by mass or more, may be 10000 ppb by mass or more, may be 50000 ppb by mass or more, and may be 100000 ppb by mass or more. In contrast, the upper limit of the content of sodium atoms in the condensed ring-containing aromatic compound prepared in the step (A-1) is not particularly restricted, and the content, for example, may be 10000000 ppb by mass or less, may be 5000000 ppb by mass or less, may be 1000000 ppb by mass or less, and may be 500000 ppb by mass or less.

As the purification method in the step (A-2), methods exemplified in <$C^1$ and $C^H$ reduction method> described above are mentioned.

The content of sodium atoms in the condensed ring-containing aromatic compound after the step (A-2) is usually 41000 ppb by mass or less, and it is preferably 4100 ppb by mass or less, more preferably 410 ppb by mass or less, further preferably 100 ppb by mass or less, particularly preferably 50 ppb by mass or less, especially preferably 10 ppb by mass or less, especially more preferably 5 ppb by mass or less, especially further preferably 1 ppb by mass or less and especially particularly preferably 0 ppb by mass since the initial deterioration of the light emitting device of the present embodiment is more suppressed.

The guest material preparation step may comprise a preparation step (B-1) of preparing an aromatic amine compound containing sodium atoms mixed therein, and a step (B-2) of purifying at least a part of the aromatic amine compound prepared in the step (B-1) to remove at least a part of sodium atoms.

The content of sodium atoms in the aromatic amine compound prepared in the step (B-1) is not particularly restricted, and for example, may be 400 ppb by mass or more, may be 420 ppb by mass or more, may be 500 ppb by mass or more, may be 1000 ppb by mass or more, may be 5000 ppb by mass or more, may be 10000 ppb by mass or more, may be 50000 ppb by mass or more, and may be 100000 ppb by mass or more. In contrast, the upper limit of the content of sodium atoms in the aromatic amine compound prepared in the step (A-1) is not particularly restricted, and the content, for example, may be 10000000 ppb by mass or less, may be 5000000 ppb by mass or less, may be 1000000 ppb by mass or less, and may be 500000 ppb by mass or less.

As the purification method in the step (B-2), for example, methods exemplified in <$C^1$ and $C^H$ reduction method> described above are mentioned.

The content of sodium atoms in the aromatic amine compound after the step (B-2) is usually 40000 ppb by mass or less, and it is preferably 4000 ppb by mass or less, more preferably 400 ppb by mass or less, further preferably 380 ppb by mass or less, particularly preferably 150 ppb by mass or less, especially preferably 50 ppb by mass or less, especially more preferably 10 ppb by mass or less, especially further preferably 1 ppb by mass or less and especially particularly preferably 0 ppb by mass since the initial deterioration of the light emitting device of the present embodiment is more suppressed.

In the production method (1), the host material and the guest material are mixed in the production step at a blending ratio such that the total amount of both is 400 ppb by mass or less in consideration of the amount of sodium atoms contained in the host material and the amount of sodium atoms contained in the guest material. By this, it is possible to obtain a composition for light emitting device with which the initial deterioration of the light emitting device can be suppressed.

The method of mixing the host material and the guest material in the production step in the production method (1) is not particularly restricted and includes, for example, a method of dissolving the host material and the guest material in the solvent explained in the section of the ink described above and mixing them, a method of mixing the host material and the guest material at the solid state, a method of mixing the host material and the guest material by co-vapor-deposition, and the like.

The production method (1) may further comprise a host material measurement step of measuring the content of sodium atoms contained in the condensed ring-containing aromatic compound. The production method (1) may further comprise a guest material measurement step of measuring the content of sodium atoms contained in the aromatic amine compound. It is preferable that the production method (1) comprises the host material measurement step and the guest material measurement step. The method for measuring the content of sodium atoms in the host material measurement

step and the guest material measurement step is preferably an ICP/MS method.

It is preferable that the host material measurement step and the guest material measurement step are carried out before the production step in the production method (1).

In the production method (1), the host material preparation step preferably comprises the host material measurement step. In the production method (1), the guest material preparation step preferably comprises the guest material measurement step.

<Production method (2)>

In another embodiment, the method for producing a composition for light emitting device may be a production method of a composition for light emitting device comprising a host material preparation step of preparing a host material containing a condensed ring-containing aromatic compound, a determination step of determining the blending ratio of the guest material to the host material, a guest material preparation step of preparing a guest material which contains an aromatic amine compound and with which, when mixed with the host material at the above-described blending ratio, the total amount of sodium atoms contained in the host material and sodium atoms contained in the guest material is 400 ppb by mass or less with respect to the total amount of the host material and the guest material, and a production step of mixing the host material and the guest material at the blending ratio to obtain a composition for light emitting device (hereinafter, referred to also as "production method (2)").

In the production method (2), the host material preparation step may comprise a step (A-1) of preparing a condensed ring-containing aromatic compound containing sodium atoms mixed therein, and a step (A-2) of purifying at least a part of the condensed ring-containing aromatic compound prepared in the step (A-1) to remove at least a part of sodium atoms. The step (A-1) and the step (A-2) in the production method (2) may be the same as the step (A-1) and the step (A-2) in the production method (1) described above.

In the production method (2), the blending ratio may be determined according to the properties of a light emitting device in the determination step. In the determination step, for example, the blending ratio may be determined based on the fabrication results of a light emitting device with a test composition using materials similar to the host material and the guest material described above, or the blending ratio may be determined based on the fabrication results of a light emitting device with a test composition in which the content of sodium atoms exceeds 400 ppb by mass.

In the guest material preparation step in the production method (2), the content of sodium atoms allowable for the guest material is determined according to the content of sodium atoms in the host material prepared in the host material preparation step and the blending ratio determined in the determination step. That is, the guest material preparation step can be said to be a step of preparing a guest material in which the content of sodium atoms is within a permissible range.

In the production method (2), the guest material preparation step may comprise, for example, a preparation step (B-1) of preparing an aromatic amine compound containing sodium atoms mixed therein, and a step (B-2) of purifying at least a part of the aromatic amine compound prepared in the step (B-1) to remove at least a part of sodium atoms. The step (B-1) and the step (B-2) in the production method (2) may be the same as the step (B-1) and the step (B-2) in the production method (1) described above.

In the production method (2), the host material prepared in the host material preparation step and the guest material prepared in the guest material preparation step are mixed, in the production step, at the blending ratio determined in the determination step. By this, it is possible to obtain a composition for light emitting device with which the initial deterioration of the light emitting device can be suppressed.

The method for mixing the host material and the guest material in the production step in the production method (2) may be the same as the method for mixing the host material and the guest material in the production step in the production method (1).

The production method (2) may further comprise the host material measurement step described above. The production method (2) may further comprise the guest material measurement step described above. It is preferable that the production method (2) comprises the host material measurement step described above and the guest material measurement step described above.

In the production method (2), it is preferable that the host material measurement step described above and the guest material measurement step described above are carried out before the production step.

In the production method (2), the host material preparation step preferably comprises the host material measurement step described above. In the production method (2), the guest material preparation step preferably comprises the guest material measurement step described above.

<Production method (3)>

In still another embodiment, the method for producing a composition for light emitting device may be a production method of a composition for light emitting device comprising a guest material preparation step of preparing a guest material

containing an aromatic amine compound, a determination step of determining the blending ratio of the host material to the guest material, a host material preparation step of preparing a host material which contains a condensed ring-containing aromatic compound and with which, when mixed the guest material at the above-described blending ratio, the total amount of sodium atoms contained in the host material and sodium atoms contained in the guest material is 400 ppb by mass or less with respect to the total amount of the host material and the guest material, and a production step of mixing the guest material and the host material at the above-described blending ratio to obtain a composition for light emitting device (hereinafter, referred to also as "production method (3)").

In the production method (3), the guest material preparation step may comprise a step (B-1) of preparing an aromatic amine compound containing sodium atoms mixed therein, and a step (B-2) of purifying at least a part of the aromatic amine compound prepared in the step (B-1) to remove at least a part of sodium atoms. The step (B-1) and the step (B-2) in the production method (3) may be the same as the step (B-1) and the step (B-2) in the production method (1)

In the production method (3), the blending ratio may be determined according to the properties of a light emitting device in the determination step. In the determination step, for example, the blending ratio may be determined based on the fabrication results of a light emitting device with a test composition using materials similar to the host material and the guest material described above, or the blending ratio may be determined based on the fabrication results of a light emitting device with a test composition in which the content of sodium atoms exceeds 400 ppb by mass.

In the host material preparation step in the production method (3), the content of sodium atoms allowable for the host material is determined according to the content of sodium atoms in the guest material prepared in the guest material preparation step and the blending ratio determined in the determination step. That is, the host material preparation step can be said to be a step of preparing the host material in which the content of sodium atoms is within a permissible range.

In the production method (3), the host material preparation step may comprise, for example, a preparation step (A-1) of preparing a condensed ring-containing aromatic compound containing sodium atoms mixed therein, and a step (A-2) of purifying at least a part of the condensed ring-containing aromatic compound prepared in the step (A-1) to remove at least a part of sodium atoms. The step (A-1) and the step (A-2) in the production method (3) may be the same as the step (A-1) and the step (A-2) in the production method (1) described above.

In the production method (3), the guest material prepared in the guest material preparation step and the host material prepared in the host material preparation step are mixed, in the production step, at the blending ratio determined in the determination step. By this, it is possible to obtain a composition for light emitting device with which the initial deterioration of the light emitting device can be suppressed.

The method for mixing the host material and the guest material in the production step in the production method (3) may be the same as the method for mixing the host material and the guest material in the production step in the production method (1).

The production method (3) may further comprise the host material measurement step described above. The production method (3) may further comprise the guest material measurement step described above. It is preferable that the production method (3) comprises the host material measurement step described above and the guest material measurement step described above.

In the production method (3), it is preferable that the host material measurement step described above and the guest material measurement step described above are carried out before the production step.

In the production method (3), the host material preparation step preferably comprises the host material measurement step described above. In the production method (3), the guest material preparation step preferably comprises the guest material measurement step described above.

<Production method (4)>

In still another embodiment, the method for producing a composition for light emitting device may be a production method of a composition for light emitting device comprising a host material preparation step of preparing a condensed ring-containing aromatic compound as the host material, a guest material preparation step of preparing an aromatic amine compound as the guest material, a determination step of determining the blending ratio of the host material and the guest material, a purification step of purifying at least a part of the condensed ring-containing aromatic compound and the aromatic amine compound with which, when the host material and the guest material are mixed at the above-described blending ratio, the total amount of sodium atoms contained in the host material and sodium atoms contained in the guest material is 400 ppb by mass or less with respect to the total amount of the host material and the guest material, and a production step of mixing the guest material containing the condensed ring-containing aromatic compound and the guest material containing the aromatic amine compound at the above-described blending ratio to obtain a composition for light emitting device (hereinafter, referred to also as "production method (4)").

In the production method (4), sodium atoms may be mixed in at least one of the condensed ring-containing aromatic compound prepared in the host material preparation step and the aromatic amine compound prepared in the guest material preparation step. That is, the host material preparation step may be a step of preparing a condensed ring-containing aromatic compound containing sodium atoms mixed therein, or the guest material preparation step may be a step of preparing an aromatic amine compound containing sodium atoms mixed therein.

In the production method (4), the blending ratio may be determined according to the properties of a light emitting device in the determination step. In the determination step, for example, the blending ratio may be determined based on the fabrication results of a light emitting device with a test composition using materials similar to the host material and the guest material described above, the blending ratio may be determined based on the fabrication results of a light emitting device with a test composition in which the content of sodium atoms exceeds 400 ppb by mass, or the blending ratio may be determined based on the fabrication results of a light emitting device with a test composition mixed with the condensed ring-containing aromatic compound and the aromatic amine compound prepared in the host material preparation step and the guest material preparation step.

In the production method (4), at least a part of the condensed ring-containing aromatic compound and the aromatic amine compound is purified in the purification step. As the purification method, methods exemplified in <$C^1$ and $C^H$ reduction methods described above are mentioned. The purification step may be a step of purifying only one of the condensed ring-containing aromatic compound and the aromatic amine compound or a step of purifying both the condensed ring-containing aromatic compound and the aromatic amine compound.

In the production method (4), the condensed ring-containing aromatic compound and the aromatic amine compound are mixed, in the production step, at the blending ratio determined in the determination step. In this procedure, the total amount of sodium atoms contained in the host material and sodium atoms contained in the guest material with respect to the total amount of the host material and the guest material is 400 ppb by mass or less, since the purification step is involved. By this, it is possible to obtain a composition for light emitting device with which the initial deterioration of a light emitting device can be suppressed.

The method for mixing the host material and the guest material in the production step in the production method (4) may be the same as the method for mixing the host material and the guest material in the production step in the production method (1).

The production method (4) may further comprise the host material measurement step described above. The production method (4) may further comprise the guest material measurement step described above. It is preferable that the production method (4) comprises the host material measurement step described above and the guest material measurement step described above.

In the production method (4), it is preferable that the host material measurement step described above and the guest material measurement step described above are carried out before the production step.

In the production method (4), it is preferable that the host material preparation step or the purification step comprises the host material measurement step described above. In the production method (2), it is preferable that the guest material preparation step or the purification step comprises the guest material measurement step described above.

Another aspect of the present invention relates to a method for producing a light emitting device. This production method may be a method for producing a light emitting device containing an anode, a cathode, and an organic layer disposed between the anode and the cathode, comprising a step of forming the organic layer with the composition for light emitting device produced by any of the production methods (1) to (4) described above.

In the method for producing a light emitting device of the present embodiment, the organic layer can be formed, for example, by using the same method as for fabrication of a film described above.

Further, in the method for producing a light emitting device of the present embodiment, the production method described in the column of <Light emitting device> described above may be used.

Furthermore, as the light emitting device in the method for producing a light emitting device of the present embodiment, for example, light emitting devices described in the column of <Light emitting device> described above are mentioned.

## EXAMPLES

The present invention will be illustrated further in detain by examples below, but the present invention is not limited to these examples.

In the present examples, the maximum peak wavelength of the emission spectrum of a compound was measured by a spectrophotometer (manufactured by JASCO Corporation, FP-6500) at room temperature. A compound was dissolved in xylene at a concentration of about $0.8\times10^{-4}$ % by mass and the resultant xylene solution was used as a sample. As the excitation light, UV light with a wavelength of 325 nm was used.

In the present examples, the amount of sodium atoms contained in a compound was measured by an ICP/MASS method.

<Compound H1 and compound EM1>

A compound H1 was synthesized with reference to a method described in Japanese Unexamined Patent Application Publication (JP-A) No. 2011-105643.
A compound EM1 was synthesized with reference to a method described in International Publication WO2011/137922.

[Chemical Formula 9]

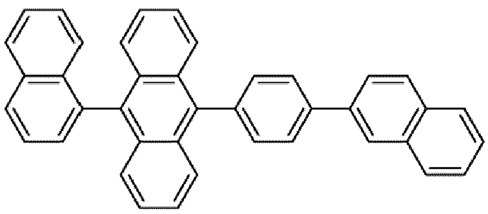

compound H1 and H2

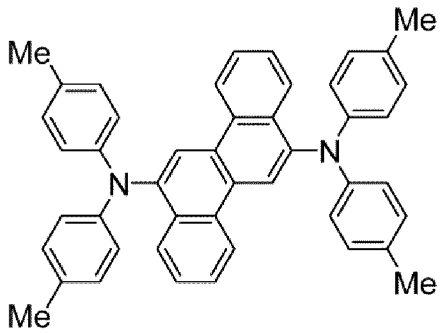

compound EM1 and EM2

The HPLC area percentage value of the compound H1 was 99.5% or more. The amount ($C^H$) of sodium atoms contained in the compound H1 was 410 ppb by mass.
The HPLC area percentage value of the compound EM1 was 99.5% or more. The amount ($C^1$) of sodium atoms contained in the compound EM1 was 420 ppb by mass.

<Purification of compound H1 (Synthesis of compound H2)>

Sublimation purification of the compound H1 was repeated until the amount of sodium atoms contained in the compound H1 became the detection limit or less (0 ppb by mass), to obtain a compound H2. In sublimation purification, the degree of vacuum was set to $3\times10^{-3}$Pa to $5\times10^{-3}$Pa, and the sublimation temperature was set to 250°C to 300°C.
The HPLC area percentage value was the compound H2 was 99.5% or more. The amount ($C^H$) of sodium atoms contained in the compound H2 was below the detection limit (0 ppb by mass).

<Purification of compound EM1 (Synthesis of compound EM2)>

Sublimation purification of the compound EM1 was repeated until the amount of sodium atoms contained in the compound EM1 became the detection limit or less (0 ppb by mass), to obtain a compound EM2. In sublimation purification, the degree of vacuum was set to $3\times10^{-3}$Pa to $5\times10^{-3}$Pa, and the sublimation temperature was set to 250°C to 300°C.
The HPLC area percentage value was the compound EM2 was 99.5% or more. The amount ($C^1$) of sodium atoms contained in the compound EM2 was below the detection limit (0 ppb by mass).
The maximum peak wavelength of the emission spectrum of the compounds H1 and H2 was 421 nm. The maximum peak wavelength of the emission spectrum of the compounds EM1 and EM2 was 454 nm.

<Example D1> Fabrication and evaluation of light emitting device D1

(Formation of anode and hole injection layer)

An ITO film was attached with a thickness of 45 nm to a glass substrate by a sputtering method, for form an anode. On the anode, a hole injection material ND-3202 (manufactured by Nissan Chemical Corporation) was spin-coated to form a film with a thickness of 35 nm. The substrate carrying the hole injection layer laminated thereon was placed under an air atmosphere and heated on a hot plate at 50°C for 3 minutes, and further, heated at 230°C for 15 minutes, to form a hole injection layer.

(Formation of hole transporting layer)

A polymer compound HTL-1 was dissolved in xylene at a concentration of 0.7% by mass. The resultant xylene

solution was spin-coated on the hole injection layer, to form a film with a thickness of 20 nm which was then placed under a nitrogen gas atmosphere and heated on a hot plate at 180°C for 60 minutes, to form a hole transporting layer. The polymer compound HTL-1 is a polymer compound of Polymer Example 1 in International Publication WO2014/102543.

(Formation of light emitting layer)

The compound H2 and the compound EM2 (compound H2/compound EM2 = 90% by mass/10% by mass) were dissolved at a concentration of 2% by mass in toluene. The resultant toluene solution was spin-coated on the hole transporting layer, to form a film with a thickness of 60 nm which was then placed under a nitrogen gas atmosphere and heated at 130°C for 10 minutes, to form a light emitting layer.

(Formation of cathode)

The substrate carrying the light emitting layer formed thereon was placed in a vapor deposition machine and the internal pressure was reduced to $1.0 \times 10^{-4}$ Pa or less, then, sodium fluoride was vapor-deposited with a thickness of about 4 nm on the light emitting layer, then, aluminum was vapor-deposited with a thickness of about 80 nm on the sodium fluoride layer, as cathodes. After vapor deposition, sealing was performed with a glass substrate, to fabricate a light emitting device D1.

(Evaluation of light emitting device)

Voltage was applied to the light emitting device D1, to observe EL emission. The current value was set so that the initial luminance was 5000 $cd/m^2$, then, the device was driven at constant current, and the time until the luminance reached 97% of the initial luminance (hereinafter, referred to also as "LT97") was measured.

<Examples D2 to D10 and Comparative Example CD1>

Fabrication and evaluation of light emitting devices D2 to D10 and CD1

Light emitting devices D2 to D10 and CD1 were fabricated in the same manner as in Example D1, except that materials described in Table 1 were used at the material ratio described in Table 1 instead of "the compound H2 and the compound EM2 (compound H2/compound EM2 = 90% by mass/10% by mass)" in (Formation of light emitting layer) of Example D1.

Voltage was applied to the light emitting devices D2 to D10 and CD1, to observe EL emission. LT97 of the light emitting devices D2 to D10 and CD1 was measured.

The results of Examples D1 to D10 and Comparative Example CD1 are shown in Table 1. The relative values of LT97 of the light emitting devices D1 to D10 if LT97 of the light emitting device CD1 is taken as 1.0 are shown.

[Table 1]

| | light emitting device | Light emitting layer | | | | | LT97 (relative value) |
|---|---|---|---|---|---|---|---|
| | | Material | Material ratio (% by mass) | $C^H$ (ppb by mass) | $C^1$ (ppb by mass) | $C^HW^H+C^1W^1$ (ppb by mass) | |
| Example D1 | D1 | H2/EM2 | 90/10 | 0 | 0 | 0 | 2.1 |
| Example D2 | D2 | H2/EM2/EM1 | 90/9/1 | 0 | 42.0 | 4.2 | 1.7 |
| Example D3 | D3 | H2/H1/EM2 | 88/2/10 | 9.1 | 0 | 8.2 | 1.9 |
| Example D4 | D4 | H2/EM2/EM1 | 90/7/3 | 0 | 126 | 12.6 | 1.9 |
| Example D5 | D5 | H2/H1/EM2 | 85.5/4.5/10 | 20.5 | 0 | 18.5 | 1.7 |
| Example D6 | D6 | H2/EM2/EM1 | 90/1/9 | 0 | 378 | 37.8 | 1.7 |
| Example D7 | D7 | H2/H1/EM2 | 72/18/10 | 82.0 | 0 | 73.8 | 1.8 |
| Example D8 | D8 | H2/H1/EM2 | 45/45/10 | 205 | 0 | 185 | 1.6 |
| Example D9 | D9 | H2/H1/EM2 | 18/72/10 | 328 | 0 | 295 | 1.8 |
| Example D10 | D10 | H1/EM2 | 90/10 | 410 | 0 | 369 | 1.8 |

(continued)

| | light emitting device | Light emitting layer | | | | | LT97 (relative value) |
|---|---|---|---|---|---|---|---|
| | | Material | Material ratio (% by mass) | $C^H$ (ppb by mass) | $C^1$ (ppb by mass) | $C^HW^H+C^1W^1$ (ppb by mass) | |
| Comparative Example CD1 | CD1 | H1/EM1 | 90/10 | 410 | 420 | 411 | 1.0 |

Industrial Applicability

According to the present invention, a composition which is useful for production of a light emitting device of which initial deterioration is suppressed is provided. The present invention is industrially useful since production of a light emitting device of which initial deterioration is suppressed has effects such as resource saving, energy saving and the like.

## Claims

**1.** A composition for light emitting device containing a host material and a guest material blended therein, wherein

said host material contains an aromatic compound having a condensed ring skeleton in which only three or more benzene rings are condensed,
said guest material contains an aromatic amine compound, and
the total amount of sodium atoms contained in said host material and sodium atoms contained in said guest material is 400 ppb by mass or less with respect to the total amount of said host material and said guest material; and wherein:

(a) said aromatic compound is a compound represented by the formula (FH):

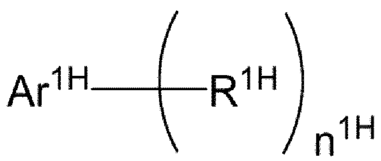

(FH)

wherein,

$n^{1H}$ represents an integer of 0 or more,
$Ar^{1H}$ represents a group obtained by removing from an aromatic hydrocarbon having a condensed ring skeleton in which only three or more benzene rings are condensed $n^{1H}$ or more hydrogen atoms bonding directly to carbon atoms constituting said condensed ring, and this group optionally has a substituent, when a plurality of the substituents are present, they may be the same or different and may be combined together to form a ring together with atoms to which they are attached,
$R^{1H}$ represents an aryl group or a monovalent heterocyclic group, and these groups optionally have a substituent, when a plurality of the substituents are present, they may be the same or different and may be combined together to form a ring together with atoms to which they are attached, when a plurality of $R^{1H}$ are present, they may be the same or different and may be combined together to form a ring together with atoms to which they are attached; and

(b) said aromatic amine compound is a compound represented by the formula (FB):

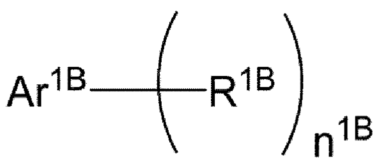

(FB)

wherein,

$n^{1B}$ represents an integer of 1 or more,
$Ar^{1B}$ represents an aromatic hydrocarbon group or an aromatic heterocyclic group, and these groups optionally have a substituent, when a plurality of the substituents are present, they may be the same or different and may be combined together to form a ring together with atoms to which they are attached,
$R^{1B}$ represents an amino group or a substituted amino group, and these groups optionally have a substituent, when a plurality of the substituents are present, they may be the same or different and may be combined together to form a ring together with atoms to which they are attached, when a plurality of $R^{1B}$ are present, they may be the same or different and may be combined together to form a ring together with atoms to which they are attached.

**2.** The composition for light emitting device according to Claim 1, wherein
said condensed ring skeleton is a condensed ring skeleton in which only three or more and five or less benzene rings are condensed.

**3.** The composition for light emitting device according to Claim 2, wherein
said condensed ring skeleton is an anthracene skeleton, a phenanthrene skeleton, a benzoanthracene skeleton, a benzophenanthrene skeleton or a pyrene skeleton.

**4.** The composition for light emitting device according to any one of Claims 1 to 3,
further comprising at least one selected from the group consisting of a hole transporting material, a hole injection material, an electron transporting material, an electron injection material, a light emitting material, an antioxidant and a solvent.

**5.** A light emitting device having an anode, a cathode, and an organic layer disposed between said anode and said cathode, wherein
said organic layer is a layer containing the composition for light emitting device as described in any one of Claims 1 to 4.

**6.** A method for producing a composition for light emitting device containing a host material and a guest material blended therein, comprising

a host material preparation step of preparing a host material containing an aromatic compound having a condensed ring skeleton in which only three or more benzene rings are condensed,
a guest material preparation step of preparing a guest material containing an aromatic amine compound, and
a production step of mixing said host material and said guest material at a blending ratio such that the total amount of sodium atoms contained in said host material and sodium atoms contained in said guest material is 400 ppb by mass or less to obtain a composition for light emitting device; and wherein:

(a) said aromatic compound is a compound represented by the formula (FH):

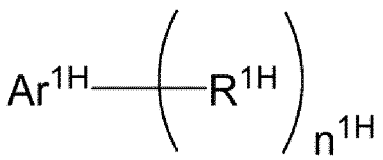
(FH)

wherein,

$n^{1H}$ represents an integer of 0 or more,
$Ar^{1H}$ represents a group obtained by removing from an aromatic hydrocarbon having a condensed ring skeleton in which only three or more benzene rings are condensed $n^{1H}$ or more hydrogen atoms bonding directly to carbon atoms constituting said condensed ring, and this group optionally has a substituent, when a plurality of the substituents are present, they may be the same or different and may be combined together to form a ring together with atoms to which they are attached,
$R^{1H}$ represents an aryl group or a monovalent heterocyclic group, and these groups optionally have a substituent, when a plurality of the substituents are present, they may be the same or different and may be combined together to form a ring together with atoms to which they are attached, when a plurality of

$R^{1H}$ are present, they may be the same or different and may be combined together to form a ring together with atoms to which they are attached; and

(b) said aromatic amine compound is a compound represented by the formula (FB):

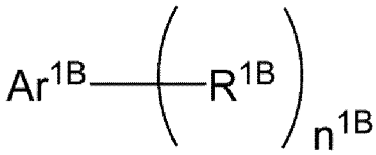 (FB)

wherein,

$n^{1B}$ represents an integer of 1 or more,
$Ar^{1B}$ represents an aromatic hydrocarbon group or an aromatic heterocyclic group, and these groups optionally have a substituent, when a plurality of the substituents are present, they may be the same or different and may be combined together to form a ring together with atoms to which they are attached,
$R^{1B}$ represents an amino group or a substituted amino group, and these groups optionally have a substituent, when a plurality of the substituents are present, they may be the same or different and may be combined together to form a ring together with atoms to which they are attached, when a plurality of $R^{1B}$ are present, they may be the same or different and may be combined together to form a ring together with atoms to which they are attached.

**7.** The production method according to Claim 6, wherein said guest material preparation step comprises

a preparation step (B-1) of preparing said aromatic amine compound containing sodium atoms mixed therein, and
a step (B-2) of purifying at least a part of said aromatic amine compound prepared in said step (B-1) to remove at least a part of said sodium atoms.

**8.** The production method according to Claim 6 or 7, wherein said host material preparation step comprises

a step (A-1) of preparing said aromatic compound containing sodium atoms mixed therein, and
a step (A-2) of purifying at least a part of said aromatic compound prepared in said step (A-1) to remove at least a part of said sodium atoms.

**9.** A method for producing a composition for light emitting device containing a host material and a guest material blended therein, comprising

a host material preparation step of preparing a host material containing an aromatic compound having a condensed ring skeleton in which only three or more benzene rings are condensed,
a determination step of determining the blending ratio of the guest material to said host material,
a guest material preparation step of preparing a guest material which contains an aromatic amine compound and with which, when mixed with said host material at said blending ratio, the total amount of sodium atoms contained in said host material and sodium atoms contained in said guest material with respect to the total amount of said host material and said guest material is 400 ppb by mass or less, and
a production step of mixing said host material and said guest material at said blending ratio to obtain a composition for light emitting device; and wherein:

(a) said aromatic compound is a compound represented by the formula (FH):

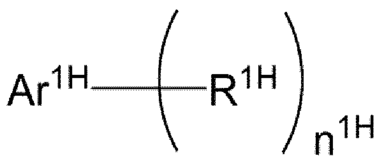 (FH)

wherein,

$n^{1H}$ represents an integer of 0 or more,
$Ar^{1H}$ represents a group obtained by removing from an aromatic hydrocarbon having a condensed ring skeleton in which only three or more benzene rings are condensed $n^{1H}$ or more hydrogen atoms bonding directly to carbon atoms constituting said condensed ring, and this group optionally has a substituent, when a plurality of the substituents are present, they may be the same or different and may be combined together to form a ring together with atoms to which they are attached,
$R^{1H}$ represents an aryl group or a monovalent heterocyclic group, and these groups optionally have a substituent, when a plurality of the substituents are present, they may be the same or different and may be combined together to form a ring together with atoms to which they are attached, when a plurality of $R^{1H}$ are present, they may be the same or different and may be combined together to form a ring together with atoms to which they are attached; and

(b) said aromatic amine compound is a compound represented by the formula (FB):

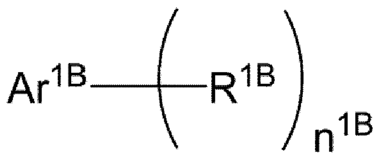
(FB)

wherein,

$n^{1B}$ represents an integer of 1 or more,
$Ar^{1B}$ represents an aromatic hydrocarbon group or an aromatic heterocyclic group, and these groups optionally have a substituent, when a plurality of the substituents are present, they may be the same or different and may be combined together to form a ring together with atoms to which they are attached,
$R^{1B}$ represents an amino group or a substituted amino group, and these groups optionally have a substituent, when a plurality of the substituents are present, they may be the same or different and may be combined together to form a ring together with atoms to which they are attached, when a plurality of $R^{1B}$ are present, they may be the same or different and may be combined together to form a ring together with atoms to which they are attached.

**10.** A method for producing a composition for light emitting device containing a host material and a guest material blended therein, comprising

a guest material preparation step of preparing a guest material containing an aromatic amine compound,
a determination step of determining the blending ratio of the host material to said guest material,
a host material preparation step of preparing a host material which contains an aromatic compound having a condensed ring skeleton in which only three or more benzene rings are condensed and with which, when mixed with said guest material at said blending ratio, the total amount of sodium atoms contained in said host material and sodium atoms contained in said guest material with respect to the total amount of said host material and said guest material is 400 ppb by mass or less, and
a production step of mixing said guest material and said host material at said blending ratio to obtain a composition for light emitting device; and wherein:

(a) said aromatic compound is a compound represented by the formula (FH):

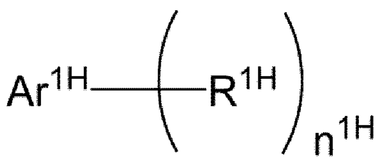
(FH)

wherein,

$n^{1H}$ represents an integer of 0 or more,
$Ar^{1H}$ represents a group obtained by removing from an aromatic hydrocarbon having a condensed ring skeleton in which only three or more benzene rings are condensed $n^{1H}$ or more hydrogen atoms bonding directly to carbon atoms constituting said condensed ring, and this group optionally has a substituent, when a plurality of the substituents are present, they may be the same or different and may be combined together to form a ring together with atoms to which they are attached,
$R^{1H}$ represents an aryl group or a monovalent heterocyclic group, and these groups optionally have a substituent, when a plurality of the substituents are present, they may be the same or different and may be combined together to form a ring together with atoms to which they are attached, when a plurality of $R^{1H}$ are present, they may be the same or different and may be combined together to form a ring together with atoms to which they are attached; and

(b) said aromatic amine compound is a compound represented by the formula (FB):

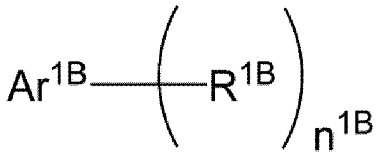

(FB)

wherein,

$n^{1B}$ represents an integer of 1 or more,
$Ar^{1B}$ represents an aromatic hydrocarbon group or an aromatic heterocyclic group, and these groups optionally have a substituent, when a plurality of the substituents are present, they may be the same or different and may be combined together to form a ring together with atoms to which they are attached,
$R^{1B}$ represents an amino group or a substituted amino group, and these groups optionally have a substituent, when a plurality of the substituents are present, they may be the same or different and may be combined together to form a ring together with atoms to which they are attached, when a plurality of $R^{1B}$ are present, they may be the same or different and may be combined together to form a ring together with atoms to which they are attached.

**11.** A method for producing a composition for light emitting device containing a host material and a guest material blended therein, comprising

a host material preparation step of preparing an aromatic compound having a condensed ring skeleton in which only three or more benzene rings are condensed as the host material,
a guest material preparation step of preparing an aromatic amine compound as the guest material,
a determination step of determining the blending ratio of said host material and said guest material,
a purification step of purifying at least a part of said aromatic compound and said aromatic amine compound such that, when said host material and said guest material are mixed at said blending ratio, the total amount of sodium atoms contained in said host material and sodium atoms contained in said guest material with respect to the total amount of said host material and said guest material is 400 ppb by mass or less, and
a production step of mixing said host material containing said aromatic compound and said guest material containing said aromatic amine compound at said blending ratio to obtain a composition for light emitting device; and wherein:

(a) said aromatic compound is a compound represented by the formula (FH):

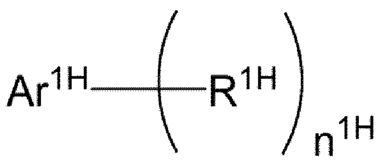

(FH)

wherein,

$n^{1H}$ represents an integer of 0 or more,
$Ar^{1H}$ represents a group obtained by removing from an aromatic hydrocarbon having a condensed ring skeleton in which only three or more benzene rings are condensed $n^{1H}$ or more hydrogen atoms bonding directly to carbon atoms constituting said condensed ring, and this group optionally has a substituent, when a plurality of the substituents are present, they may be the same or different and may be combined together to form a ring together with atoms to which they are attached,
$R^{1H}$ represents an aryl group or a monovalent heterocyclic group, and these groups optionally have a substituent, when a plurality of the substituents are present, they may be the same or different and may be combined together to form a ring together with atoms to which they are attached, when a plurality of $R^{1H}$ are present, they may be the same or different and may be combined together to form a ring together with atoms to which they are attached; and

(b) said aromatic amine compound is a compound represented by the formula (FB):

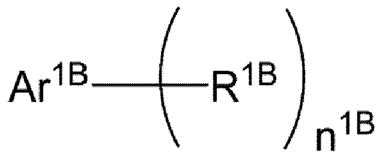
(FB)

wherein,

$n^{1B}$ represents an integer of 1 or more,
$Ar^{1B}$ represents an aromatic hydrocarbon group or an aromatic heterocyclic group, and these groups optionally have a substituent, when a plurality of the substituents are present, they may be the same or different and may be combined together to form a ring together with atoms to which they are attached,
$R^{1B}$ represents an amino group or a substituted amino group, and these groups optionally have a substituent, when a plurality of the substituents are present, they may be the same or different and may be combined together to form a ring together with atoms to which they are attached, when a plurality of $R^{1B}$ are present, they may be the same or different and may be combined together to form a ring together with atoms to which they are attached.

**12.** The production method according to any one of Claims 6 to 11, further comprising

a host material measurement step of measuring the content of sodium atoms contained in said aromatic compound, and
a guest material measurement step of measuring the content of sodium atoms contained in said aromatic amine compound.

**13.** A method for producing a light emitting device containing an anode, a cathode, and an organic layer disposed between said anode and said cathode, comprising
a step of forming said organic layer with the composition for light emitting device produced by the production method as described in any one of Claims 6 to 12.

**Patentansprüche**

**1.** Zusammensetzung für eine lichtemittierende Vorrichtung, die ein Wirtsmaterial und ein darin eingemischtes Gastmaterial enthält, wobei

das Wirtsmaterial eine aromatische Verbindung mit einem kondensierten Ringskelett enthält, in dem nur drei oder mehr Benzolringe kondensiert sind,
das Gastmaterial eine aromatische Aminverbindung enthält, und
die Gesamtmenge an Natriumatomen, die in dem Wirtsmaterial enthalten ist, und an Natriumatomen, die in dem Gastmaterial enthalten ist, 400 ppb nach Masse oder weniger beträgt, bezogen auf die Gesamtmenge des Wirtsmaterials und des Gastmaterials; und wobei:

(a) die aromatische Verbindung eine Verbindung ist, die durch die Formel (FH) dargestellt wird:

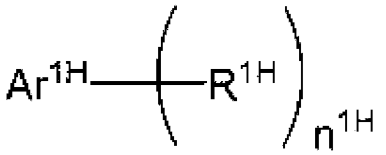 (FH)

wobei,

$n^{1H}$ eine ganze Zahl von 0 oder mehr darstellt,
$Ar^{1H}$ eine Gruppe darstellt, die durch Entfernen von einem aromatischen Kohlenwasserstoff mit einem kondensierten Ringskelett, in dem nur drei oder mehr Benzolringe kondensiert sind, erhalten wird, wobei $n^{1H}$ oder mehr Wasserstoffatome direkt an Kohlenstoffatome gebunden sind, die den kondensierten Ring bilden, und diese Gruppe optional einen Substituenten aufweist, wobei, wenn eine Vielzahl von Substituenten vorhanden ist, diese gleich oder verschieden sein können und zusammen mit den Atomen, an die sie gebunden sind, einen Ring bilden können,
$R^{1H}$ eine Arylgruppe oder eine einwertige heterocyclische Gruppe darstellt, und diese Gruppen optional einen Substituenten aufweisen, wenn eine Vielzahl der Substituenten vorhanden ist, können sie gleich oder verschieden sein und können zusammen mit Atomen, an die sie gebunden sind, einen Ring bilden, wenn eine Vielzahl von $R^{1H}$ vorhanden ist, können sie gleich oder verschieden sein und können zusammen mit Atomen, an die sie gebunden sind, einen Ring bilden; und

(b) die aromatische Aminverbindung eine Verbindung ist, die durch die Formel (FB) dargestellt wird:

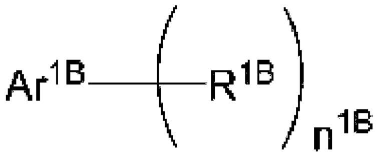 (FB)

wobei,

$n^{1B}$ eine ganze Zahl von 1 oder mehr darstellt,
$Ar^{1B}$ eine aromatische Kohlenwasserstoffgruppe oder eine aromatische heterocyclische Gruppe darstellt, und diese Gruppen optional einen Substituenten aufweisen, wobei, wenn eine Vielzahl von Substituenten vorhanden ist, diese gleich oder verschieden sein können und zusammen mit den Atomen, an die sie gebunden sind, einen Ring bilden können,
$R^{1B}$ eine Aminogruppe oder eine substituierte Aminogruppe darstellt, und diese Gruppen optional einen Substituenten aufweisen, wenn eine Vielzahl der Substituenten vorhanden ist, können sie gleich oder verschieden sein und können zusammen mit Atomen, an die sie gebunden sind, einen Ring bilden, wenn eine Vielzahl von $R^{1B}$ vorhanden ist, können sie gleich oder verschieden sein und können zusammen mit Atomen, an die sie gebunden sind, einen Ring bilden.

**2.** Zusammensetzung für eine lichtemittierende Vorrichtung nach Anspruch 1, wobei
das kondensierte Ringskelett ein kondensiertes Ringskelett ist, in dem nur drei oder mehr und fünf oder weniger Benzolringe kondensiert sind.

**3.** Zusammensetzung für eine lichtemittierende Vorrichtung nach Anspruch 2, wobei
das kondensierte Ringskelett ein Anthracenskelett, ein Phenanthrenskelett, ein Benzoanthracenskelett, ein Benzophenanthrenskelett oder ein Pyrenskelett ist.

**4.** Zusammensetzung für eine lichtemittierende Vorrichtung nach einem der Ansprüche 1 bis 3,
die ferner mindestens ein Material umfasst, das aus der Gruppe ausgewählt ist, die aus einem Lochtransportmaterial, einem Lochinjektionsmaterial, einem Elektronentransportmaterial, einem Elektroneninjektionsmaterial, einem lichtemittierenden Material, einem Antioxidationsmittel und einem Lösungsmittel besteht.

**5.** Lichtemittierende Vorrichtung mit einer Anode, einer Kathode und einer organischen Schicht, die zwischen der Anode und der Kathode angeordnet ist, wobei

die organische Schicht eine Schicht ist, die die in einem der Ansprüche 1 bis 4 beschriebene Zusammensetzung für eine lichtemittierende Vorrichtung enthält.

**6.** Verfahren zur Herstellung einer Zusammensetzung für eine lichtemittierende Vorrichtung, die ein Wirtsmaterial und ein darin eingemischtes Gastmaterial enthält, das Folgendes umfasst

einen Schritt zur Herstellung des Wirtsmaterials, bei dem ein Wirtsmaterial hergestellt wird, das eine aromatische Verbindung mit einem kondensierten Ringskelett enthält, in dem nur drei oder mehr Benzolringe kondensiert sind,
einen Schritt zur Herstellung eines Gastmaterials, bei dem ein Gastmaterial hergestellt wird, das eine aromatische Aminverbindung enthält, und
einen Herstellungsschritt des Mischens des Wirtsmaterials und des Gastmaterials in einem Mischungsverhältnis, so dass die Gesamtmenge an Natriumatomen, die in dem Wirtsmaterial enthalten ist, und Natriumatomen, die in dem Gastmaterial enthalten ist, 400 ppb nach Masse oder weniger beträgt, um eine Zusammensetzung für eine lichtemittierende Vorrichtung zu erhalten; und wobei:

(a) die aromatische Verbindung eine Verbindung ist, die durch die Formel (FH) dargestellt wird:

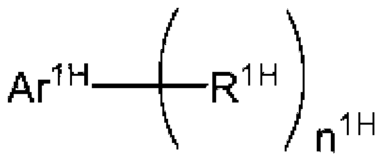

(FH)

wobei,

$n^{1H}$ eine ganze Zahl von 0 oder mehr darstellt,
$Ar^{1H}$ eine Gruppe darstellt, die durch Entfernen von einem aromatischen Kohlenwasserstoff mit einem kondensierten Ringskelett, in dem nur drei oder mehr Benzolringe kondensiert sind, erhalten wird, $n^{1H}$ oder mehr Wasserstoffatome, die direkt an Kohlenstoffatome gebunden sind, die den kondensierten Ring bilden, und diese Gruppe optional einen Substituenten aufweist, wobei, wenn eine Vielzahl von Substituenten vorhanden ist, diese gleich oder verschieden sein können und zusammen mit den Atomen, an die sie gebunden sind, einen Ring bilden können,
$R^{1H}$ eine Arylgruppe oder eine einwertige heterocyclische Gruppe darstellt, und diese Gruppen optional einen Substituenten aufweisen, wenn eine Vielzahl der Substituenten vorhanden ist, können sie gleich oder verschieden sein und können zusammen mit Atomen, an die sie gebunden sind, einen Ring bilden, wenn eine Vielzahl von $R^{1H}$ vorhanden ist, können sie gleich oder verschieden sein und können zusammen mit Atomen, an die sie gebunden sind, einen Ring bilden; und

(b) die aromatische Aminverbindung eine Verbindung ist, die durch die Formel (FB) dargestellt wird:

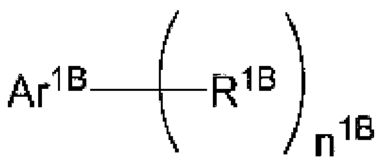

(FB)

wobei,

$n^{1B}$ eine ganze Zahl von 1 oder mehr darstellt,
$Ar^{1B}$ eine aromatische Kohlenwasserstoffgruppe oder eine aromatische heterocyclische Gruppe darstellt, und diese Gruppen optional einen Substituenten aufweisen, wenn eine Vielzahl der Substituenten vorhanden ist, können sie gleich oder verschieden sein und können zusammen mit Atomen, an die sie gebunden sind, einen Ring bilden,
$R^{1B}$ eine Aminogruppe oder eine substituierte Aminogruppe darstellt, und diese Gruppen optional einen Substituenten aufweisen, wenn eine Vielzahl der Substituenten vorhanden ist, können sie gleich oder verschieden sein und können zusammen mit Atomen, an die sie gebunden sind, einen Ring bilden, wenn

eine Vielzahl von $R^{1B}$ vorhanden ist, können sie gleich oder verschieden sein und können zusammen mit Atomen, an die sie gebunden sind, einen Ring bilden.

**7.** Herstellungsverfahren nach Anspruch 6, wobei der Schritt der Herstellung des Gastmaterials Folgendes umfasst:

einen Herstellungsschritt (B-1) der Herstellung der aromatischen Aminverbindung, die Natriumatome in Mischung enthält, und
einen Schritt (B-2) des Reinigens mindestens eines Teils der in Schritt (B-1) hergestellten aromatischen Aminverbindung, um mindestens einen Teil der Natriumatome zu entfernen.

**8.** Herstellungsverfahren nach Anspruch 6 oder 7, wobei der Schritt der Herstellung des Wirtsmaterials Folgendes umfasst

einen Schritt (A-1) der Herstellung der aromatischen Verbindung umfasst, die Natriumatome im Gemisch enthält, und
einen Schritt (A-2) des Reinigens mindestens eines Teils der in Schritt (B-1) hergestellten aromatischen Aminverbindung, um mindestens einen Teil der Natriumatome zu entfernen.

**9.** Verfahren zur Herstellung einer Zusammensetzung für eine lichtemittierende Vorrichtung, die ein Wirtsmaterial und ein darin eingemischtes Gastmaterial enthält, das Folgendes umfasst:

einen Schritt zur Herstellung des Wirtsmaterials, bei dem ein Wirtsmaterial hergestellt wird, das eine aromatische Verbindung mit einem kondensierten Ringskelett enthält, in dem nur drei oder mehr Benzolringe kondensiert sind,
einen Bestimmungsschritt zum Bestimmen des Mischungsverhältnisses des Gastmaterials zum Wirtsmaterial,
einen Schritt der Herstellung des Gastmaterials, das eine aromatische Aminverbindung enthält und bei dem, wenn es mit dem Wirtsmaterial in dem Mischungsverhältnis gemischt wird, die Gesamtmenge der im Wirtsmaterial enthaltenen Natriumatome und der im Gastmaterial enthaltenen Natriumatome, bezogen auf die Gesamtmenge des Wirtsmaterials und des Gastmaterials, 400 ppb nach Masse oder weniger beträgt, und
einen Herstellungsschritt des Mischens des Wirtsmaterials und des Gastmaterials in dem Mischungsverhältnis, um eine Zusammensetzung für eine lichtemittierende Vorrichtung zu erhalten; und wobei:

(a) die aromatische Verbindung eine Verbindung ist, die durch die Formel (FH) dargestellt wird:

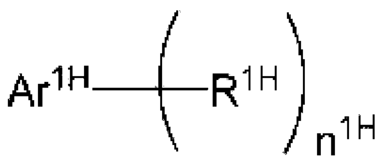

(FH)

wobei,

$n^{1H}$ eine ganze Zahl von 0 oder mehr darstellt,
$Ar^{1H}$ eine Gruppe darstellt, die durch Entfernen von einem aromatischen Kohlenwasserstoff mit einem kondensierten Ringskelett, in dem nur drei oder mehr Benzolringe kondensiert sind, erhalten wird, $n^{1H}$ oder mehr Wasserstoffatome, die direkt an Kohlenstoffatome gebunden sind, die den kondensierten Ring bilden, und diese Gruppe optional einen Substituenten aufweist, wenn eine Vielzahl der Substituenten vorhanden ist, können sie gleich oder verschieden sein und können zusammen mit Atomen, an die sie gebunden sind, einen Ring bilden,
$R^{1H}$ eine Arylgruppe oder eine einwertige heterocyclische Gruppe darstellt, und diese Gruppen optional einen Substituenten aufweisen, wenn eine Vielzahl der Substituenten vorhanden ist, können sie gleich oder verschieden sein und können zusammen mit Atomen, an die sie gebunden sind, einen Ring bilden, wenn eine Vielzahl von $R^{1H}$ vorhanden ist, können sie gleich oder verschieden sein und können zusammen mit Atomen, an die sie gebunden sind, einen Ring bilden; und

(b) die aromatische Aminverbindung eine Verbindung ist, die durch die Formel (FB) dargestellt wird:

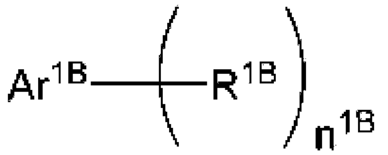 (FB)

wobei,

$n^{1B}$ eine ganze Zahl von 1 oder mehr darstellt,
$Ar^{1B}$ eine aromatische Kohlenwasserstoffgruppe oder eine aromatische heterocyclische Gruppe darstellt, und diese Gruppen optional einen Substituenten aufweisen, wenn eine Vielzahl der Substituenten vorhanden ist, können sie gleich oder verschieden sein und können zusammen mit Atomen, an die sie gebunden sind, einen Ring bilden,
$R^{1B}$ eine Aminogruppe oder eine substituierte Aminogruppe darstellt, und diese Gruppen optional einen Substituenten aufweisen, wenn eine Vielzahl der Substituenten vorhanden ist, können sie gleich oder verschieden sein und können zusammen mit Atomen, an die sie gebunden sind, einen Ring bilden, wenn eine Vielzahl von $R^{1B}$ vorhanden ist, können sie gleich oder verschieden sein und können zusammen mit Atomen, an die sie gebunden sind, einen Ring bilden.

**10.** Verfahren zur Herstellung einer Zusammensetzung für eine lichtemittierende Vorrichtung, die ein Wirtsmaterial und ein darin eingemischtes Gastmaterial enthält, das Folgendes umfasst

einen Schritt zur Herstellung eines Gastmaterials, bei dem ein Gastmaterial hergestellt wird, das eine aromatische Aminverbindung enthält,
einen Bestimmungsschritt des Bestimmens des Mischungsverhältnisses des Wirtsmaterials zum Gastmaterial,
einen Schritt zur Herstellung des Wirtsmaterials, bei dem ein Wirtsmaterial hergestellt wird, das eine aromatische Verbindung mit einem kondensierten Ringskelett enthält, in dem nur drei oder mehr Benzolringe kondensiert sind, und bei dem, wenn es mit dem Gastmaterial im Mischungsverhältnis gemischt wird, die Gesamtmenge der im Wirtsmaterial enthaltenen Natriumatome und der im Gastmaterial enthaltenen Natriumatome, bezogen auf die Gesamtmenge des Wirtsmaterials und des Gastmaterials, 400 ppb nach Masse oder weniger beträgt, und
einen Herstellungsschritt des Mischens des Gastmaterials und des Wirtsmaterials in dem Mischungsverhältnis, um eine Zusammensetzung für eine lichtemittierende Vorrichtung zu erhalten; und wobei:

(a) die aromatische Verbindung eine Verbindung ist, die durch die Formel (FH) dargestellt wird:

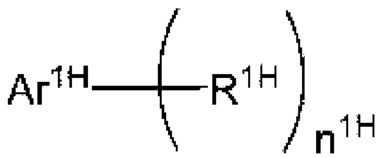 (FH)

wobei,

$n^{1H}$ eine ganze Zahl von 0 oder mehr darstellt,
$Ar^{1H}$ eine Gruppe darstellt, die durch Entfernen von einem aromatischen Kohlenwasserstoff mit einem kondensierten Ringskelett, in dem nur drei oder mehr Benzolringe kondensiert sind, erhalten wird, $n^{1H}$ oder mehr Wasserstoffatome, die direkt an Kohlenstoffatome gebunden sind, die den kondensierten Ring bilden, und diese Gruppe optional einen Substituenten aufweist, wenn eine Vielzahl der Substituenten vorhanden ist, können sie gleich oder verschieden sein und können zusammen mit Atomen, an die sie gebunden sind, einen Ring bilden,
$R^{1H}$ eine Arylgruppe oder eine einwertige heterocyclische Gruppe darstellt, und diese Gruppen optional einen Substituenten aufweisen, wenn eine Vielzahl der Substituenten vorhanden ist, können sie gleich oder verschieden sein und können zusammen mit Atomen, an die sie gebunden sind, einen Ring bilden, wenn eine Vielzahl von $R^{1H}$ vorhanden ist, können sie gleich oder verschieden sein und können zusammen mit Atomen, an die sie gebunden sind, einen Ring bilden; und

(b) die aromatische Aminverbindung eine Verbindung ist, die durch die Formel (FB) dargestellt wird:

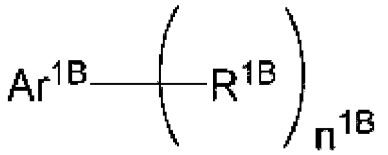 (FB)

wobei,

$n^{1B}$ eine ganze Zahl von 1 oder mehr darstellt,
$Ar^{1B}$ eine aromatische Kohlenwasserstoffgruppe oder eine aromatische heterocyclische Gruppe darstellt, und diese Gruppen optional einen Substituenten aufweisen, wenn eine Vielzahl der Substituenten vorhanden ist, können sie gleich oder verschieden sein und können zusammen mit Atomen, an die sie gebunden sind, einen Ring bilden,
$R^{1B}$ eine Aminogruppe oder eine substituierte Aminogruppe darstellt, und diese Gruppen optional einen Substituenten aufweisen, wenn eine Vielzahl der Substituenten vorhanden ist, können sie gleich oder verschieden sein und können zusammen mit Atomen, an die sie gebunden sind, einen Ring bilden, wenn eine Vielzahl von $R^{1B}$ vorhanden ist, können sie gleich oder verschieden sein und können zusammen mit Atomen, an die sie gebunden sind, einen Ring bilden.

**11.** Verfahren zur Herstellung einer Zusammensetzung für eine lichtemittierende Vorrichtung, die ein Wirtsmaterial und ein darin eingemischtes Gastmaterial enthält, das Folgendes umfasst

einen Schritt zur Herstellung des Wirtsmaterials, bei dem eine aromatische Verbindung mit einem kondensierten Ringskelett, in dem nur drei oder mehr Benzolringe kondensiert sind, als Wirtsmaterial hergestellt wird,
einen Schritt zur Herstellung eines Gastmaterials, bei dem eine aromatische Aminverbindung als Gastmaterial hergestellt wird,
einen Bestimmungsschritt des Bestimmens des Mischungsverhältnisses des Wirtsmaterials und des Gastmaterials,
einen Reinigungsschritt, bei dem mindestens ein Teil der aromatischen Verbindung und der aromatischen Aminverbindung so gereinigt wird, dass, wenn das Wirtsmaterial und das Gastmaterial in dem Mischungsverhältnis gemischt werden, die Gesamtmenge der im Wirtsmaterial enthaltenen Natriumatome und der im Gastmaterial enthaltenen Natriumatome, bezogen auf die Gesamtmenge des Wirtsmaterials und des Gastmaterials, 400 ppb nach Masse oder weniger beträgt, und
einen Herstellungsschritt des Mischens des Wirtsmaterials, das die aromatische Verbindung enthält, und des Gastmaterials, das die aromatische Aminverbindung enthält, in dem Mischungsverhältnis, um eine Zusammensetzung für eine lichtemittierende Vorrichtung zu erhalten; und wobei:

(a) die aromatische Verbindung eine Verbindung ist, die durch die Formel (FH) dargestellt wird:

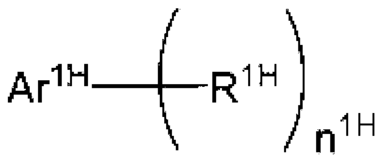 (FH)

wobei,

$n^{1H}$ eine ganze Zahl von 0 oder mehr darstellt,
$Ar^{1H}$ eine Gruppe darstellt, die durch Entfernen von einem aromatischen Kohlenwasserstoff mit einem kondensierten Ringskelett, in dem nur drei oder mehr Benzolringe kondensiert sind, erhalten wird, $n^{1H}$ oder mehr Wasserstoffatome, die direkt an Kohlenstoffatome gebunden sind, die den kondensierten Ring bilden, und diese Gruppe optional einen Substituenten aufweist, wenn eine Vielzahl der Substituenten vorhanden ist, können sie gleich oder verschieden sein und können zusammen mit Atomen, an die sie gebunden sind, einen Ring bilden,
$R^{1H}$ eine Arylgruppe oder eine einwertige heterocyclische Gruppe darstellt, und diese Gruppen optional einen Substituenten aufweisen, wenn eine Vielzahl der Substituenten vorhanden ist, können sie gleich oder verschieden sein und können zusammen mit Atomen, an die sie gebunden sind, einen Ring bilden,

wenn eine Vielzahl von $R^{1H}$ vorhanden ist, können sie gleich oder verschieden sein und können zusammen mit Atomen, an die sie gebunden sind, einen Ring bilden; und

(b) die aromatische Aminverbindung eine Verbindung ist, die durch die Formel (FB) dargestellt wird:

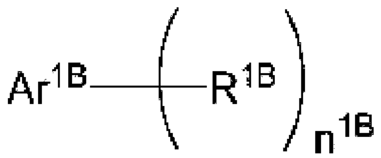 (FB)

wobei,

$n^{1B}$ eine ganze Zahl von 1 oder mehr darstellt,
$Ar^{1B}$ eine aromatische Kohlenwasserstoffgruppe oder eine aromatische heterocyclische Gruppe darstellt, und diese Gruppen optional einen Substituenten aufweisen, wenn eine Vielzahl der Substituenten vorhanden ist, können sie gleich oder verschieden sein und können zusammen mit Atomen, an die sie gebunden sind, einen Ring bilden,
$R^{1B}$ eine Aminogruppe oder eine substituierte Aminogruppe darstellt, und diese Gruppen optional einen Substituenten aufweisen, wenn eine Vielzahl der Substituenten vorhanden ist, können sie gleich oder verschieden sein und können zusammen mit Atomen, an die sie gebunden sind, einen Ring bilden, wenn eine Vielzahl von $R^{1B}$ vorhanden ist, können sie gleich oder verschieden sein und können zusammen mit Atomen, an die sie gebunden sind, einen Ring bilden.

**12.** Herstellungsverfahren nach einem der Ansprüche 6 bis 11, das ferner folgendes umfasst:

einen Schritt zum Messen des Wirtsmaterials, bei dem der Gehalt an Natriumatomen, die in der aromatischen Verbindung enthalten sind, gemessen wird, und
einen Schritt zum Messen des Gastmaterials, bei dem der Gehalt an Natriumatomen, die in der aromatischen Aminverbindung enthalten sind, gemessen wird.

**13.** Verfahren zur Herstellung einer lichtemittierenden Vorrichtung, die eine Anode, eine Kathode und eine zwischen der Anode und der Kathode angeordnete organische Schicht enthält, das umfasst:
einen Schritt des Bildens der organischen Schicht mit der Zusammensetzung für die lichtemittierende Vorrichtung, die durch das in einem der Ansprüche 6 bis 12 beschriebene Herstellungsverfahren hergestellt wird.

## Revendications

**1.** Composition pour dispositif luminescent contenant un matériau hôte et un matériau invité mélangés dans celle-ci, dans laquelle

ledit matériau hôte contient un composé aromatique ayant un squelette annulaire condensé dans lequel seulement trois ou plus de trois anneaux benzène sont condensés,
ledit matériau invité contient un composé amine aromatique, et
la quantité totale d'atomes de sodium contenus dans ledit matériau hôte et d'atomes de sodium contenus dans ledit matériau invité est de 400 ppb en masse ou moins par rapport à la quantité totale dudit matériau hôte et dudit matériau invité ; et dans laquelle :

(a) ledit composé aromatique est un composé représenté par la formule (FH) :

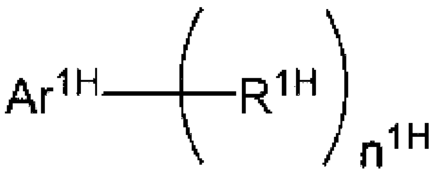 (FH)

où,

$n^{1H}$ représentant un nombre entier de 0 ou de plus de 0,
$Ar^{1H}$ représentant un groupe obtenu en éliminant, d'un hydrocarbure aromatique ayant un squelette annulaire condensé dans lequel seulement trois ou plus de trois anneaux benzène sont condensés, $n^{1H}$ ou plus de $n^{1H}$ atomes d'hydrogène se liant directement à des atomes de carbone constituant ledit anneau condensé, et ce groupe a facultativement un substituant, lorsqu'une pluralité des substituants sont présents, ils peuvent être les mêmes ou différents et peuvent être combinés ensemble pour former un anneau conjointement avec des atomes auxquels ils sont attachés,
$R^{1H}$ représentant un groupe aryle ou un groupe hétérocyclique monovalent, et ces groupes ont facultativement un substituant, lorsqu'une pluralité des substituants sont présents, ils peuvent être les mêmes ou différents et peuvent être combinés ensemble pour former un anneau conjointement avec des atomes auxquels ils sont attachés, lorsqu'une pluralité de $R^{1H}$ sont présents, ils peuvent être les mêmes ou différents et peuvent être combinés ensemble pour former un anneau conjointement avec des atomes auxquels ils sont attachés ; et

(b) ledit composé amine aromatique est un composé représenté par la formule (FB) :

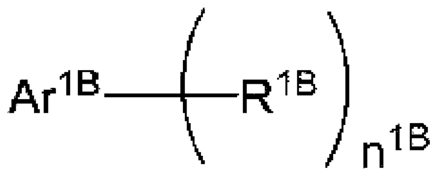

(FB)

où,

$n^{1B}$ représentant un nombre entier de 1 ou de plus de 1,
$Ar^{1B}$ représentant un hydrocarbure aromatique groupe ou un groupe hétérocyclique aromatique, et ces groupes ont facultativement un substituant, lorsqu'une pluralité des substituants sont présents, ils peuvent être les mêmes ou différents et peuvent être combinés ensemble pour former un anneau conjointement avec des atomes auxquels ils sont attachés,
$R^{1B}$ représentant un groupe amino ou un groupe amino substitué, et ces groupes ont facultativement un substituant, lorsqu'une pluralité des substituants sont présents, ils peuvent être les mêmes ou différents et peuvent être combinés ensemble pour former un anneau conjointement avec des atomes auxquels ils sont attachés, lorsqu'une pluralité de $R^{1B}$ sont présents, ils peuvent être les mêmes ou différents et peuvent être combinés ensemble pour former un anneau conjointement avec des atomes auxquels ils sont attachés.

**2.** Composition pour dispositif luminescent selon la revendication 1, dans laquelle ledit squelette annulaire condensé est un squelette annulaire condensé dans lequel seulement trois ou plus de trois et cinq ou moins de cinq anneaux benzène sont condensés.

**3.** Composition pour dispositif luminescent selon la revendication 2, dans laquelle ledit squelette annulaire condensé est un squelette anthracène, un squelette phénanthrène, un squelette benzoanthracène, un squelette benzophénanthrène, ou un squelette pyrène.

**4.** Composition pour dispositif luminescent selon l'une quelconque des revendications 1 à 3, comprenant en outre au moins un élément sélectionné parmi le groupe constitué de : un matériau de transport de trous, un matériau d'injection de trous, un matériau de transport d'électrons, un matériau d'injection d'électrons, un matériau luminescent, un antioxydant, et un solvant.

**5.** Dispositif luminescent ayant une anode, une cathode, et une couche organique disposée entre ladite anode et ladite cathode, dans lequel ladite couche organique est une couche contenant la composition pour dispositif luminescent telle que décrite dans l'une quelconque des revendications 1 à 4.

**6.** Procédé pour produire une composition pour dispositif luminescent contenant un matériau hôte et un matériau invité

mélangés dans celle-ci, comprenant

une étape de préparation de matériau hôte, de la préparation d'un matériau hôte contenant un composé aromatique ayant un squelette annulaire condensé dans lequel seulement trois ou plus de trois anneaux benzène sont condensés,
une étape de préparation de matériau invité, de la préparation d'un matériau invité contenant un composé amine aromatique, et
une étape de production, du mélangeage dudit matériau hôte et dudit matériau invité à un a rapport de mélangeage de manière telle que la quantité totale d'atomes de sodium contenus dans ledit matériau hôte et d'atomes de sodium contenus dans ledit matériau invité est de 400 ppb en masse ou moins pour obtenir une composition pour dispositif luminescent ; et dans lequel :

(a) ledit composé aromatique est un composé représenté par la formule (FH) :

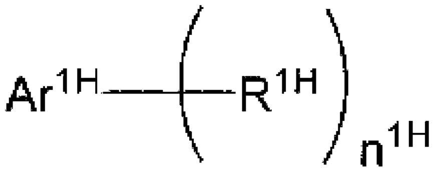

(FH)

où,

$n^{1H}$ représentant un nombre entier de 0 ou de plus de 0,
$Ar^{1H}$ représentant un groupe obtenu en éliminant, d'un hydrocarbure aromatique ayant un squelette annulaire condensé dans lequel seulement trois ou plus de trois anneaux benzène sont condensés, $n^{1H}$ ou plus de $n^{1H}$ atomes d'hydrogène se liant directement à des atomes de carbone constituant ledit anneau condensé, et ce groupe a facultativement un substituant, lorsqu'une pluralité des substituants sont présents, ils peuvent être les mêmes ou différents et peuvent être combinés ensemble pour former un anneau conjointement avec des atomes auxquels ils sont attachés,
$R^{1H}$ représentant un groupe aryle ou un groupe hétérocyclique monovalent, et ces groupes ont facultativement un substituant, lorsqu'une pluralité des substituants sont présents, ils peuvent être les mêmes ou différents et peuvent être combinés ensemble pour former un anneau conjointement avec des atomes auxquels ils sont attachés, lorsqu'une pluralité de $R^{1H}$ sont présents, ils peuvent être les mêmes ou différents et peuvent être combinés ensemble pour former un anneau conjointement avec des atomes auxquels ils sont attachés ; et

(b) ledit composé amine aromatique est un composé représenté par la formule (FB)

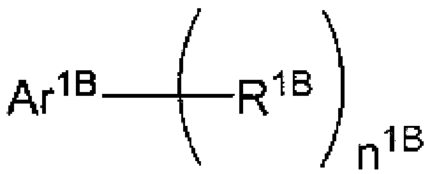

(FB)

où,

$n^{1B}$ représentant un nombre entier de 1 ou de plus de 1,
$Ar^{1B}$ représentant un hydrocarbure aromatique groupe ou un groupe hétérocyclique aromatique, et ces groupes ont facultativement un substituant, lorsqu'une pluralité des substituants sont présents, ils peuvent être les mêmes ou différents et peuvent être combinés ensemble pour former un anneau conjointement avec des atomes auxquels ils sont attachés,
$R^{1B}$ représentant un groupe amino ou un groupe amino substitué, et ces groupes ont facultativement un substituant, lorsqu'une pluralité des substituants sont présents, ils peuvent être les mêmes ou différents et peuvent être combinés ensemble pour former un anneau conjointement avec des atomes auxquels ils sont attachés, lorsqu'une pluralité de $R^{1B}$ sont présents, ils peuvent être les mêmes ou différents et peuvent être combinés ensemble pour former un anneau conjointement avec des atomes auxquels ils

sont attachés.

**7.** Procédé de production selon la revendication 6, dans lequel ladite étape de préparation de matériau invité comprend

une étape de préparation (B-1), de la préparation dudit composé amine aromatique contenant des atomes de sodium mélangés dans celui-ci, et
une étape (B-2) de la purification d'au moins une partie dudit composé amine aromatique préparé dans ladite étape (B-1) pour éliminer au moins une partie desdits atomes de sodium.

**8.** Procédé de production selon la revendication 6 ou 7, dans lequel ladite étape de préparation de matériau hôte comprend

une étape (A-1), de la préparation dudit composé aromatique contenant des atomes de sodium mélangés dans celui-ci, et
une étape (A-2) de la purification d'au moins une partie dudit composé aromatique préparé dans ladite étape (A-1) pour éliminer au moins une partie desdits atomes de sodium.

**9.** Procédé pour produire une composition pour dispositif luminescent contenant un matériau hôte et un matériau invité mélangés dans celle-ci, comprenant

une étape de préparation de matériau hôte, de la préparation d'un matériau hôte contenant un composé aromatique ayant un squelette annulaire condensé dans lequel seulement trois ou plus de trois anneaux benzène sont condensés,
une étape de détermination, de la détermination du rapport de mélangeage du matériau invité par rapport audit matériau hôte,
une étape de préparation de matériau invité, de la préparation d'un matériau invité qui contient un composé amine aromatique et avec lequel, lorsqu'il est mélangé avec ledit matériau hôte audit rapport de mélangeage, la quantité totale d'atomes de sodium contenus dans ledit matériau hôte et d'atomes de sodium contenus dans ledit matériau invité par rapport à la quantité totale dudit matériau hôte et dudit matériau invité est de 400 ppb en masse ou moins, et
une étape de production, du mélangeage dudit matériau hôte et dudit matériau invité audit rapport de mélangeage pour obtenir une composition pour dispositif luminescent ; et dans lequel :

(a) ledit composé aromatique est un composé représenté par la formule (FH) :

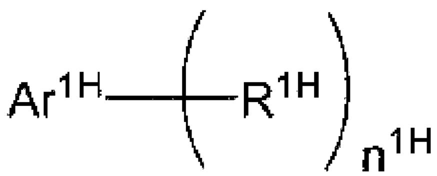

(FH)

où,

$n^{1H}$ représentant un nombre entier de 0 ou de plus de 0,
$Ar^{1H}$ représentant un groupe obtenu en éliminant, d'un hydrocarbure aromatique ayant un squelette annulaire condensé dans lequel seulement trois ou plus de trois anneaux benzène sont condensés, $n^{1H}$ ou plus de $n^{1H}$ atomes d'hydrogène se liant directement à des atomes de carbone constituant ledit anneau condensé, et ce groupe a facultativement un substituant, lorsqu'une pluralité des substituants sont présents, ils peuvent être les mêmes ou différents et peuvent être combinés ensemble pour former un anneau conjointement avec des atomes auxquels ils sont attachés,
$R^{1H}$ représentant un groupe aryle ou un groupe hétérocyclique monovalent, et ces groupes ont facultativement un substituant, lorsqu'une pluralité des substituants sont présents, ils peuvent être les mêmes ou différents et peuvent être combinés ensemble pour former un anneau conjointement avec des atomes auxquels ils sont attachés, lorsqu'une pluralité de $R^{1H}$ sont présents, ils peuvent être les mêmes ou différents et peuvent être combinés ensemble pour former un anneau conjointement avec des atomes auxquels ils sont attachés ; et

(b) ledit composé amine aromatique est un composé représenté par la formule (FB) :

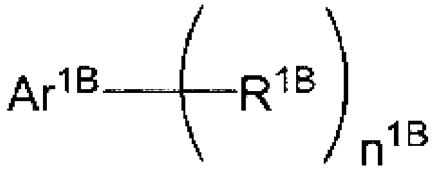

(FB)

où,

$n^{1B}$ représentant un nombre entier de 1 ou de plus de 1,
$Ar^{1B}$ représentant un hydrocarbure aromatique groupe ou un groupe hétérocyclique aromatique, et ces groupes ont facultativement un substituant, lorsqu'une pluralité des substituants sont présents, ils peuvent être les mêmes ou différents et peuvent être combinés ensemble pour former un anneau conjointement avec des atomes auxquels ils sont attachés,
$R^{1B}$ représentant un groupe amino ou un groupe amino substitué, et ces groupes ont facultativement un substituant, lorsqu'une pluralité des substituants sont présents, ils peuvent être les mêmes ou différents et peuvent être combinés ensemble pour former un anneau conjointement avec des atomes auxquels ils sont attachés, lorsqu'une pluralité de $R^{1B}$ sont présents, ils peuvent être les mêmes ou différents et peuvent être combinés ensemble pour former un anneau conjointement avec des atomes auxquels ils sont attachés.

**10.** Procédé pour produire une composition pour dispositif luminescent contenant un matériau hôte et un matériau invité mélangés dans celle-ci, comprenant

une étape de préparation de matériau invité, de la préparation d'un matériau invité contenant un composé amine aromatique,
une étape de détermination, de la détermination du rapport de mélangeage du matériau hôte par rapport audit matériau invité,
une étape de préparation de matériau hôte, de la préparation d'un matériau hôte qui contient un composé aromatique ayant un squelette annulaire condensé dans lequel seulement trois ou plus de trois anneaux benzène sont condensés et avec lequel, lorsqu'il est mélangé avec ledit matériau invité audit rapport de mélangeage, la quantité totale d'atomes de sodium contenus dans ledit matériau hôte et d'atomes de sodium contenus dans ledit matériau invité par rapport à la quantité totale dudit matériau hôte et dudit matériau invité est de 400 ppb en masse ou moins, et
une étape de production, du mélangeage dudit matériau invité et dudit matériau hôte audit rapport de mélangeage pour obtenir une composition pour dispositif luminescent ; et dans lequel :

(a) ledit composé aromatique est un composé représenté par la formule (FH) :

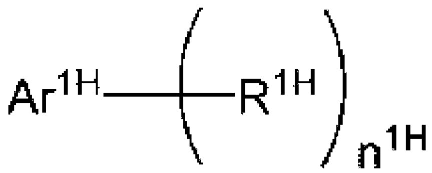

(FH)

où,

$n^{1H}$ représentant un nombre entier de 0 ou de plus de 0,
$Ar^{1H}$ représentant un groupe obtenu en éliminant, d'un hydrocarbure aromatique ayant un squelette annulaire condensé dans lequel seulement trois ou plus de trois anneaux benzène sont condensés, $n^{1H}$ ou plus de $n^{1H}$ atomes d'hydrogène se liant directement à des atomes de carbone constituant ledit anneau condensé, et ce groupe a facultativement un substituant, lorsqu'une pluralité des substituants sont présents, ils peuvent être les mêmes ou différents et peuvent être combinés ensemble pour former un anneau conjointement avec des atomes auxquels ils sont attachés,
$R^{1H}$ représentant un groupe aryle ou un groupe hétérocyclique monovalent, et ces groupes ont

facultativement un substituant, lorsqu'une pluralité des substituants sont présents, ils peuvent être les mêmes ou différents et peuvent être combinés ensemble pour former un anneau conjointement avec des atomes auxquels ils sont attachés, lorsqu'une pluralité de $R^{1H}$ sont présents, ils peuvent être les mêmes ou différents et peuvent être combinés ensemble pour former un anneau conjointement avec des atomes auxquels ils sont attachés ; et

(b) ledit composé amine aromatique est un composé représenté par la formule (FB) :

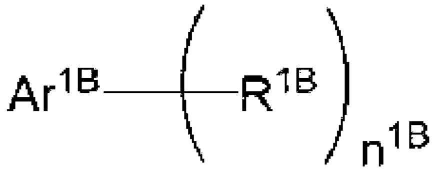

(FB)

où,

$n^{1B}$ représentant un nombre entier de 1 ou de plus de 1,
$Ar^{1B}$ représentant un hydrocarbure aromatique groupe ou un groupe hétérocyclique aromatique, et ces groupes ont facultativement un substituant, lorsqu'une pluralité des substituants sont présents, ils peuvent être les mêmes ou différents et peuvent être combinés ensemble pour former un anneau conjointement avec des atomes auxquels ils sont attachés,
$R^{1B}$ représentant un groupe amino ou un groupe amino substitué, et ces groupes ont facultativement un substituant, lorsqu'une pluralité des substituants sont présents, ils peuvent être les mêmes ou différents et peuvent être combinés ensemble pour former un anneau conjointement avec des atomes auxquels ils sont attachés, lorsqu'une pluralité de $R^{1B}$ sont présents, ils peuvent être les mêmes ou différents et peuvent être combinés ensemble pour former un anneau conjointement avec des atomes auxquels ils sont attachés.

**11.** Procédé pour produire une composition pour dispositif luminescent contenant un matériau hôte et un matériau invité mélangés dans celle-ci, comprenant

une étape de préparation de matériau hôte, de la préparation d'un composé aromatique, ayant un squelette annulaire condensé dans lequel seulement trois ou plus de trois anneaux benzène sont condensés, en tant que matériau hôte,
une étape de préparation de matériau invité, de la préparation d'un composé amine aromatique en tant que matériau invité,
une étape de détermination, de la détermination du rapport de mélangeage dudit matériau hôte et dudit matériau invité,
une étape de purification, de la purification d'au moins une partie dudit composé aromatique et dudit composé amine aromatique de manière telle que, lorsque ledit matériau hôte et ledit matériau invité sont mélangés audit rapport de mélangeage, la quantité totale d'atomes de sodium contenus dans ledit matériau hôte et d'atomes de sodium contenus dans ledit matériau invité par rapport à la quantité totale dudit matériau hôte et dudit matériau invité est de 400 ppb en masse ou moins, et
une étape de production, du mélangeage dudit matériau hôte contenant ledit composé aromatique et dudit matériau invité contenant ledit composé amine aromatique audit rapport de mélangeage pour obtenir une composition pour dispositif luminescent ; et dans lequel :

(a) ledit composé aromatique est un composé représenté par la formule (FH) :

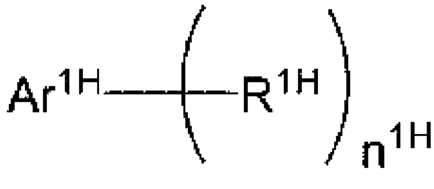

(FH)

où,

$n^{1H}$ représentant un nombre entier de 0 ou de plus de 0,
$Ar^{1H}$ représentant un groupe obtenu en éliminant, d'un hydrocarbure aromatique ayant un squelette annulaire condensé dans lequel seulement trois ou plus de trois anneaux benzène sont condensés, $n^{1H}$ ou plus de $n^{1H}$ atomes d'hydrogène se liant directement à des atomes de carbone constituant ledit anneau condensé, et ce groupe a facultativement un substituant, lorsqu'une pluralité des substituants sont présents, ils peuvent être les mêmes ou différents et peuvent être combinés ensemble pour former un anneau conjointement avec des atomes auxquels ils sont attachés,
$R^{1H}$ représentant un groupe aryle ou un groupe hétérocyclique monovalent, et ces groupes ont facultativement un substituant, lorsqu'une pluralité des substituants sont présents, ils peuvent être les mêmes ou différents et peuvent être combinés ensemble pour former un anneau conjointement avec des atomes auxquels ils sont attachés, lorsqu'une pluralité de $R^{1H}$ sont présents, ils peuvent être les mêmes ou différents et peuvent être combinés ensemble pour former un anneau conjointement avec des atomes auxquels ils sont attachés ; et

(b) ledit composé amine aromatique est un composé représenté par la formule (FB) :

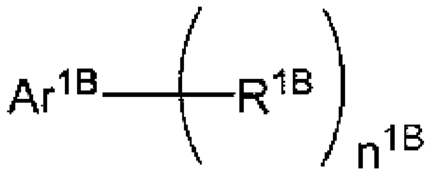

(FB)

où,

$n^{1B}$ représentant un nombre entier de 1 ou de plus de 1,
$Ar^{1B}$ représentant un hydrocarbure aromatique groupe ou un groupe hétérocyclique aromatique, et ces groupes ont facultativement un substituant, lorsqu'une pluralité des substituants sont présents, ils peuvent être les mêmes ou différents et peuvent être combinés ensemble pour former un anneau conjointement avec des atomes auxquels ils sont attachés,
$R^{1B}$ représentant un groupe amino ou un groupe amino substitué, et ces groupes ont facultativement un substituant, lorsqu'une pluralité des substituants sont présents, ils peuvent être les mêmes ou différents et peuvent être combinés ensemble pour former un anneau conjointement avec des atomes auxquels ils sont attachés, lorsqu'une pluralité de $R^{1B}$ sont présents, ils peuvent être les mêmes ou différents et peuvent être combinés ensemble pour former un anneau conjointement avec des atomes auxquels ils sont attachés.

**12.** Procédé de production selon l'une quelconque des revendications 6 à 11, comprenant en outre

une étape de mesure de matériau hôte, de la mesure de la teneur en atomes de sodium contenus dans ledit composé aromatique, et
une étape de mesure de matériau invité, de la mesure de la teneur en atomes de sodium contenus dans ledit composé amine aromatique.

**13.** Procédé pour produire un dispositif luminescent contenant une anode, une cathode, et une couche organique disposée entre ladite anode et ladite cathode, comprenant
une étape de formation de ladite couche organique avec la composition pour dispositif luminescent produite par le procédé de production tel que décrit dans l'une quelconque des revendications 6 à 12.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017170313 A
- US 2008113468 A
- JP 2011105643 A
- WO 2011137922 A
- WO 2014102543 A


### Non-patent literature cited in the description


- **MARUZEN**. 4th Edition Experimental Chemistry Course. 1993
- **MARUZEN**. 5th Edition Experimental Chemistry Course. 2007
- **MARUZEN**. *New Experimental Chemistry Course*, 1975
- Guide to Organic Chemical Experiment. Kagaku-Dojin Publishing Company, Inc., 1988